(19) **Europäisches Patentamt / European Patent Office / Office européen des brevets**

(11) **EP 4 299 850 B1**

(12) **EUROPEAN PATENT SPECIFICATION**

(45) Date of publication and mention
of the grant of the patent:
**10.06.2026 Bulletin 2026/24**

(21) Application number: **22206550.0**

(22) Date of filing: **10.11.2022**

(51) International Patent Classification (IPC):
**G16H 50/30** (2018.01)

(52) Cooperative Patent Classification (CPC):
**E03B 3/34; G16H 30/20; G16H 40/67; G16H 50/30;
G16H 50/50; G16H 50/70**

(54) **GROUNDWATER POLLUTANT CONTROL METHOD AND APPARATUS BASED ON SINGLE-WELL PUMPING-INJECTION**

VERFAHREN UND VORRICHTUNG ZUR KONTROLLE VON GRUNDWASSERSCHADSTOFFEN BASIEREND AUF PUMPEINSPRITZUNG MIT EINER EINZIGEN BOHRUNG

PROCÉDÉ ET APPAREIL DE RÉGULATION DE POLLUANTS D'EAUX SOUTERRAINES BASÉS SUR UNE INJECTION PAR POMPAGE À PUITS UNIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **01.07.2022 CN 202210764267**

(43) Date of publication of application:
**03.01.2024 Bulletin 2024/01**

(73) Proprietor: **China Three Gorges Co., Ltd.
Wuhan Hubei 430010 (CN)**

(72) Inventors:
• **LIU, Xiaoting**
  **Wuhan, 430010 (CN)**
• **DAI, Huichao**
  **Wuhan, 430010 (CN)**
• **SUN, Hongguang**
  **Wuhan, 430010 (CN)**

• **LIU, Zhiwei**
  **Wuhan, 430010 (CN)**
• **JIANG, Dingguo**
  **Wuhan, 430010 (CN)**
• **ZHAI, Yanwei**
  **Wuhan, 430010 (CN)**
• **ZHAO, Hanqing**
  **Wuhan, 430010 (CN)**
• **ZHANG, Chengxiao**
  **Wuhan, 430010 (CN)**
• **YIN, Zhaokai**
  **Wuhan, 430010 (CN)**

(74) Representative: **J A Kemp LLP
80 Turnmill Street
London EC1M 5QU (GB)**

(56) References cited:
**CN-A- 104 671 385    CN-A- 110 146 332**

Description

TECHNICAL FIELD

The present disclosure relates to the field of environment control, in particular to a groundwater pollutant control method and apparatus based on single-well pumping-injection.

BACKGROUND

At present, a single-well pumping-injection process widely exists in a utilization process of groundwater, for example, a pollutant, such as industrial wastewater, enters an artesian aquifer (aquifer) via a deep well and is diffused with the movement of the groundwater, and in a groundwater pollutant control process based on single-well pumping-injection during groundwater pollutant control, physical cleaning may also be performed by adopting a water pumping way. However, due to a non-homogeneous structure of an underground medium, it is very difficult to accurately describe the transport rule of the groundwater pollutant, namely a groundwater solute according to the Fick's law; and by the existing means, it is difficult to know about a situation caused after the groundwater pollutant is diffused, and thus, various parameters for water pumping in a control stage cannot be accurately specified. Therefore, how to effectively draw up a corresponding control plan for the groundwater pollutant has become an important issue to be solved in the industry at present. CN 104671385 A discloses a soil remediation wherein a single-well pumping-injection process is utilised.

SUMMARY

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes. Embodiments of the present disclosure provide a groundwater pollutant control method and apparatus based on single-well pumping-injection to solve the current problem that various parameters for water pumping in a control stage cannot be accurately specified due to difficulty in knowing about a situation caused after a groundwater pollutant is diffused.

According to a first aspect, an embodiment of the present disclosure provides a groundwater pollutant control method based on single-well pumping-injection. The method includes the steps of independent claim 1.

In combination with the first aspect, in a first implementation of the first aspect, the step of determining the first parameters, second parameters and third parameters of a to-be-controlled area includes:

determining the third parameters on the basis of exploration data of the to-be-controlled area; the third parameters including water quality parameters reflecting inhomogeneity and anisotropy; and
determining the first parameters and the second parameters; the first parameters including a starting time, a duration, a water injection speed, and a water injection volume of the water injection stage; and the second parameters being a duration of the standing stage.

In combination with the first aspect, in a third implementation of the first aspect, the pumping-injection three-stage solute transport model is trained on the basis of the sample water quality parameters of the aquifer in the sample area and second sample pumping-injection parameters collected for a water tracer injected into the sample area in the water injection stage, the standing stage and the water pumping stage of the aquifer;
accordingly, the pumping-injection three-stage solute transport model is established further by the following steps:

injecting the water tracer into the sample area, and determining the second sample pumping-injection parameters; the second sample pumping-injection parameters including a starting time, a duration, a water injection speed and a water injection volume of the tracer of the water injection stage, a duration of the standing stage as well as a starting time, a duration and a water pumping speed of the water pumping stage; and
using the sample water quality parameters and the second sample pumping-injection parameters as parameters used for establishment to establish the pumping-injection three-stage solute transport model; the pumping-injection three-stage solute transport model being a fractional derivative equation established under polar coordinates by stage-dividing on the basis of three stages including the water injection stage, the standing stage and the water pumping stage.

In combination with the second implementation of the first aspect, in a fifth implementation of the first aspect, the pumping-injection three-stage solute transport model is further trained on the basis of working condition parameters of the sample area; the working condition parameters include an initial concentration and boundary conditions of the groundwater pollutant;

accordingly, the pumping-injection three-stage solute transport model is established by the following steps:

determining the sample area on the basis of the third parameters;

determining the sample water quality parameters and the working condition parameters of the sample area;

determining the first sample pumping-injection parameters; the first sample pumping-injection parameters including the starting time, the duration, the water injection speed and the water injection volume of the water injection stage, the duration of the standing stage as well as the starting time, the duration and the water pumping speed of the water pumping stage; and

using the sample water quality parameters, the first sample pumping-injection parameters and the working condition parameters as parameters used for establishment to establish the pumping-injection three-stage solute transport model; the pumping-injection three-stage solute transport model being a fractional derivative equation established under polar coordinates by stage-dividing on the basis of three stages including the water injection stage, the standing stage and the water pumping stage.

In combination with the fifth implementation of the first aspect, in a sixth implementation of the first aspect, the pumping-injection three-stage solute transport model is:

$$
\begin{cases}
\frac{\partial C(r,t)}{\partial t} + \beta_0 \, {}^{TC}_0 D_t^{\alpha_0, \lambda_0} C(r,t) = -\frac{v_0}{r} \frac{\partial C(r,t)}{\partial r} + \frac{D_0}{r} \frac{\partial^2 C(r,t)}{\partial r^2}, t \in [0, T_1] \\
\frac{\partial C(r,t)}{\partial t} + \beta_1 \, {}^{TC}_{T_1} D_t^{\alpha_1, \lambda_1} C(r,t) = \frac{D_1}{r} \frac{\partial^2 C(r,t)}{\partial r^2}, t \in [T_1, T_2] \\
\frac{\partial C(r,t)}{\partial t} + \beta_2 \, {}^{TC}_{T_2} D_t^{\alpha_2, \lambda_2} C(r,t) = -\frac{v_2}{r} \frac{\partial C(r,t)}{\partial r} + \frac{D_2}{r} \, {}^{RL}_0 D_r^{\gamma} C(x,t), t \in [T_2, T_3] \\
\frac{\partial C(r,t)}{\partial r} \big|_{\partial \Omega} = 0 \\
C(r,0) = \begin{cases} A, r = 0 \\ 0, else \end{cases}, C(r, T_1^+) = C(r, T_1^-), C(r, T_2^+) = C(r, T_2^-)
\end{cases}
$$

wherein r represents a transport radius of the groundwater pollutant; t represents a transport time of the groundwater pollutant; C represents a concentration of the groundwater pollutant; $[0, T_1]$ represents the duration of the water injection stage; $[T_1, T_2]$ represents the duration of the standing stage; $[T_2, T_3]$ represents the duration of the water pumping stage; $T_3$ represents the sum of the durations of the water injection stage, the standing stage and the water pumping stage; $\beta_0$ represents a fractional-order capacity coefficient of the water injection stage; $\beta_1$ represents a fractional-order capacity coefficient of the standing stage; $\beta_2$ represents a fractional-order capacity coefficient of the water pumping stage; $\lambda_0$ represents a truncation coefficient of the water injection stage; $\lambda_1$ represents a truncation coefficient of the standing stage; $\lambda_2$ represents a truncation coefficient of the water pumping stage; $\alpha_0$ represents a fractional order of the water injection stage; $\alpha_1$ represents a fractional order of the standing stage; $\alpha_2$ represents a temporal fractional order of the water pumping stage; $\gamma$ represents a spatial fractional order of the water pumping stage, $0 < \alpha_0, \alpha_1, \alpha_2 \leq 1, 1 < \gamma \leq 2, \alpha_0, \alpha_1, \alpha_2$ and $\gamma$ are all determined on the basis of the inhomogeneity of the sample water quality parameters and are in negative correlation with the inhomogeneity of the sample water quality parameters as well as the inhomogeneity of each of the sample water quality parameters; $D_0$ represents a diffusion parameter of the water injection stage; $D_1$ represents a diffusion parameter of the standing stage; $D_2$ represents a diffusion parameter of the water pumping stage; $v_0$ represents a flow parameter of the water injection stage, $v_2$ represents a flow parameter of the water pumping stage, $v_0 > 0$, $v_2 < 0$, $A$ represents an initial concentration of the groundwater pollutant, and $\partial \Omega$ represents derivative calculation along a boundary.

According to a second aspect, an embodiment of the present disclosure further provides a groundwater pollutant control apparatus based on single-well pumping-injection, in accordance with independent claim 6.

According to a third aspect, an embodiment of the present disclosure further provides an electronic device, including a memory, a processor, and a computer program stored in the memory and capable of running on the processor, wherein the processor, when executing the program, implements the steps of the above any groundwater pollutant control method based on single-well pumping-injection.

According to a fourth aspect, an embodiment of the present disclosure further provides a non-transitory computer-readable storage medium, with a computer program stored therein, wherein the computer program, when executed by a processor, implements the steps of the above any groundwater pollutant control method based on single-well pumping-injection.

According to the groundwater pollutant control method and apparatus based on single-well pumping-injection and the device provided by the present disclosure, based on the first two physical stages of the single-well pumping-injection and geological characteristics of the to-be-controlled area, and particularly based on the hydraulic parameters, capable of reflecting the inhomogeneity and anisotropy of an underground medium in the to-be-controlled area to a certain extent, of the aquifer in the to-be-controlled area, a transport process of the groundwater pollutant, namely a groundwater solute, is simulated by means of the pumping-injection three-stage solute transport model to know about a distribution situation of the groundwater pollutant in the to-be-controlled area, based on which the pollution level of groundwater is evaluated. Various parameters of the groundwater pollutant in the third stage of the single-well pumping-injection are predicted according to the distribution situation predicted by the pumping-injection three-stage solute transport model, and then, specific parameters (indexes) required for physical cleaning in the to-be-controlled area are set, so that the control plan is more applicable to a physical structure of the underground medium in the to-be-controlled area, and can better evaluate and remediate groundwater pollution, be beneficial to the application of groundwater dynamics in production and life, and provide corresponding support for evaluation and remediation of the groundwater pollution.

## BRIEF DESCRIPTION OF THE DRAWINGS

With reference to the accompanying drawings, features and advantages of the present disclosure will be understood more clearly, and the accompanying drawings are illustrative, but should not be understood as any limitations on the present disclosure. In the accompanying drawings:

Fig. 1 shows a flow chart of a groundwater pollutant control method based on single-well pumping-injection provided by the present disclosure;
Fig. 2 shows a specific flow chart of step S101 in the groundwater pollutant control method based on single-well pumping-injection provided by the present disclosure;
Fig. 3 shows a first flow chart of establishment of a pumping-injection three-stage solute transport model in the groundwater pollutant control method based on single-well pumping-injection provided by the present disclosure;
Fig. 4 shows a second flow chart of establishment of the pumping-injection three-stage solute transport model in the groundwater pollutant control method based on single-well pumping-injection provided by the present disclosure;
Fig. 5 shows a third flow chart of establishment of the pumping-injection three-stage solute transport model in the groundwater pollutant control method based on single-well pumping-injection provided by the present disclosure;
Fig. 6 shows a schematic diagram of an experimental condition of a CH3 observation well in the groundwater pollutant control method based on single-well pumping-injection provided by the present disclosure;
Fig. 7 shows a schematic diagram of part of parameters in the CH3 observation well in the groundwater pollutant control method based on single-well pumping-injection provided by the present disclosure;
Fig. 8 shows a schematic diagram of an experimental condition of a CH10 observation well in the groundwater pollutant control method based on single-well pumping-injection provided by the present disclosure;
Fig. 9 shows a schematic diagram of part of parameters in the CH10 observation well in the groundwater pollutant control method based on single-well pumping-injection provided by the present disclosure;
Fig. 10 shows a schematic diagram of a model simulation result of the CH3 observation well in the groundwater pollutant control method based on single-well pumping-injection provided by the present disclosure;
Fig. 11 shows a schematic diagram of a model simulation result of the CH10 observation well in the groundwater pollutant control method based on single-well pumping-injection provided by the present disclosure;
Fig. 12 shows a schematic structural diagram of a groundwater pollutant control apparatus based on single-well pumping-injection provided by the present disclosure; and
Fig. 13 shows a schematic structural diagram of an electronic device provided by the present disclosure.

## DETAILED DESCRIPTION

In order to make the objectives, technical solutions and advantages of embodiments of the present disclosure clearer, a clear and complete description of the technical solutions in the embodiments of the present disclosure will be given below in combination with the accompanying drawings in the embodiments of the present disclosure. Apparently, the described embodiments are a part, but not all, of the embodiments of the present disclosure.

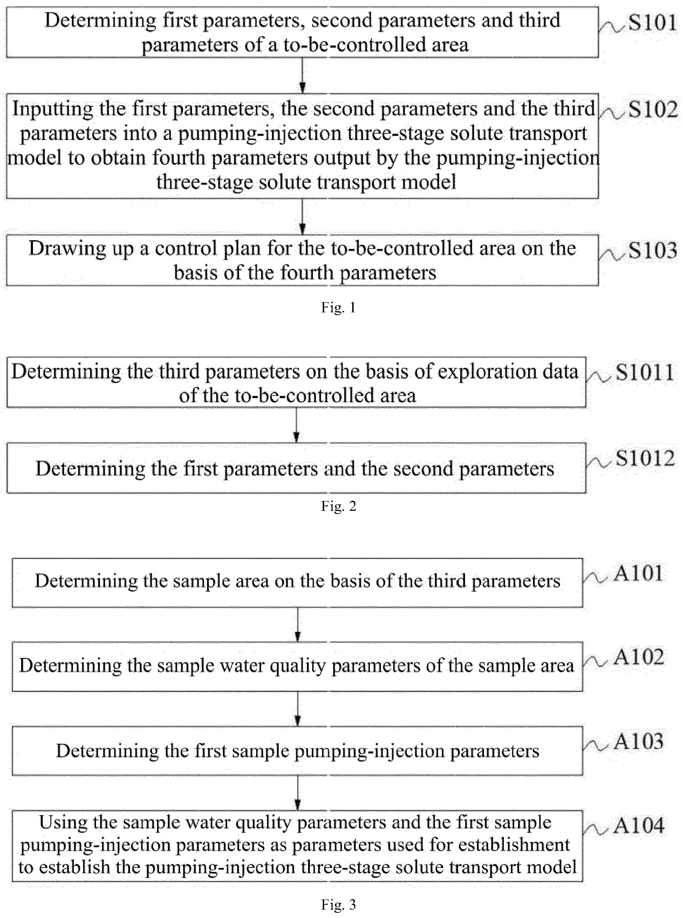

A groundwater pollutant control method based on single-well pumping-injection provided by the present disclosure will be described below in combination with Fig. 1, and the present disclosure is implemented on the basis of a single-well pumping-injection process. The method includes the following steps.

S101, first parameters, second parameters and third parameters of a to-be-controlled area are determined.

In the present embodiment, the first parameters characterize parameters of a groundwater pollutant in the to-be-controlled area in a water injection stage, the second parameters characterize parameters of the groundwater pollutant in a standing stage, and the third parameters characterize hydraulic parameters of an aquifer in the to-be-controlled area.

A single-well pumping-injection process is mainly divided into three stages, i.e., a water injection stage, a standing stage and a water pumping stage, and the water injection stage, the standing stage and the water pumping stage are performed in sequence, that is, the standing stage begins after the water injection stage ends, and the water pumping stage begins after the standing stage ends.

In a transport process of a complex medium, the flow direction of a fluid and a distribution situation of a pollutant are determined by a structure of an inhomogeneous aquifer. A range of a permeability coefficient of the same stratum may have a difference reaching up to several orders of magnitudes under complex geological conditions, the groundwater pollutant is rapidly diffused in an area where the permeability coefficient is higher and is slowly migrated and diffused in an area where the permeability coefficient is relatively low. Moreover, when the distribution situation of the groundwater pollutant in the stratum is not clear, a blind area is easily caused by existing drilling-sampling surveyance, and therefore, it is difficult to accurately recognize the situation that a site is polluted, which seriously affects the precise repair and control of the site. In the present disclosure, the third parameters include water quality parameters reflecting inhomogeneity and anisotropy. By knowing about the inhomogeneity and the anisotropy in the to-be-controlled area, the transport process of the groundwater pollutant can be better simulated, and the corresponding transport rule can be known.

Preferably, the third parameters may include the hydraulic parameters such as a permeability coefficient, a water storage coefficient, a coefficient of transmissibility, a storativity and a specific yield.

S102, the first parameters, the second parameters and the third parameters are input into a pumping-injection three-stage solute transport model to obtain fourth parameters output by the pumping-injection three-stage solute transport model.

In the present disclosure, the pumping-injection three-stage solute transport model is trained on the basis of sample water quality parameters of an aquifer in a sample area and first sample pumping-injection parameters collected for a groundwater pollutant in the sample area in the water injection stage, the standing stage and a water pumping stage.

In the present embodiment, the first parameters include a starting time, a duration, a water injection speed and a water injection volume of the water injection stage; the second parameters are a duration of the standing stage; and the fourth parameters characterize parameters of the groundwater pollutant in the water pumping stage and include a starting time, a duration and a water pumping speed of the water pumping stage.

Due to memory characteristics, a fractional-order model may depict a retention phenomenon brought by clay or fracture in the transport process of the pollutant and has been widely applied to the field of groundwater solute transport stimulation at present. However, it is difficult for an existing fractional-order model to depict a solute transport rule of the groundwater pollutant in an underground medium in the three physical processes including the water injection stage, the standing stage and the water pumping stage.

The pumping-injection three-stage solute transport model established and adopted in the present disclosure describes, depicts and simulates the three physical stages including the water injection stage, the standing stage and the water pumping stage alone at intervals, i.e., by stage-dividing, and is used for describing the transient change of the size and direction of a hydraulic gradient occurring in the three different hydraulic stages including the water injection stage, the standing stage and the water pumping stage, so that the transport rule of the pollutant solute obtained by simulation is more precise, and the more precise distribution situation of the groundwater pollutant in the to-be-controlled area can be obtained by simulation.

More specifically, the pumping-injection three-stage solute transport model is a fractional derivative equation established under polar coordinates by stage-dividing on the basis of three stages including the water injection stage, the standing stage and the water pumping stage.

S103, a control plan for the to-be-controlled area is drawn up on the basis of the fourth parameters. By using the control plan, corresponding control on groundwater pollution is performed.

According to the groundwater pollutant control method based on single-well pumping-injection provided by the present disclosure, based on the first two physical stages of the single-well pumping-injection and geological characteristics of the to-be-controlled area, and particularly based on the hydraulic parameters, capable of reflecting the inhomogeneity and anisotropy of an underground medium in the to-be-controlled area to a certain extent, of the aquifer in the to-be-controlled area, a transport process of the groundwater pollutant, namely a groundwater solute, is simulated by means of the pumping-injection three-stage solute transport model to know about a distribution situation of the groundwater pollutant in the to-be-controlled area, based on which the pollution level of groundwater is evaluated. Various parameters of the groundwater pollutant in the third stage of the single-well pumping-injection are predicted according to the distribution situation predicted by the pumping-injection three-stage solute transport model, and then, specific parameters (indexes) required for physical cleaning in the to-be-controlled area are set, so that the control plan is more applicable to a physical structure of the underground medium in the to-be-controlled area, and can better evaluate and remediate groundwater pollution, be beneficial to the application of groundwater dynamics in production and life, and provide corresponding support for evaluation and remediation of the groundwater pollution.

The groundwater pollutant control method based on single-well pumping-injection provided by the present disclosure will be described below in combination with Fig. 2. Step S101 specifically includes the following steps.

S1011, the third parameters are determined on the basis of exploration data of the to-be-controlled area. In some possible embodiments, by collecting existing data of research areas or performing relevant geological surveyance such as a single-well pumping-injection experiment, the hydraulic parameters of the aquifer in the to-be-controlled area in different directions are measured, and the geological structure of this area is analyzed, wherein the characteristic of the anisotropy of the aquifer is mainly analyzed.

S1012, the first parameters and the second parameters are determined. Various index parameters existing when a to-be-controlled area is put into the to-be-controlled area are recorded to obtain the first parameters and the second parameters required in the present disclosure.

The groundwater pollutant control method based on single-well pumping-injection provided by the present disclosure will be described below in combination with Fig. 3. The pumping-injection three-stage solute transport model in the present disclosure is established by the following steps.

A101, the sample area is determined on the basis of the third parameters.

In the present disclosure, when the sample area is screened, based on the third parameters which have been obtained and the known parameters of the underground medium structure as a candidate area, the candidate area similar to the underground medium structure in the to-be-controlled area is selected as the sample area. It can be understood that at least one candidate area can be selected as the sample area in the present disclosure.

A102, the sample water quality parameters of the sample area are determined. Similarly, the sample water quality parameters are the water quality parameters reflecting inhomogeneity and anisotropy. Values of the sample water quality parameters may slightly differ from values of the third parameters. Specifically, the specific values of the parameters may be different according to the selected sample area, but the same underground medium structure can be reflected. For example, each parameter of the sample water quality parameters in the sample area is each of the corresponding third parameters $\pm$ a preset value. Preferably, the preset value may be 3%.

A103, the first sample pumping-injection parameters are determined.

In the present disclosure, the first sample pumping-injection parameters include a starting time, a duration, a water injection speed and a water injection volume of the groundwater pollutant of the water injection stage, a duration of the standing stage as well as the starting time, the duration and the water pumping speed of the water pumping stage.

A104, the sample water quality parameters and the first sample pumping-injection parameters are used as parameters used for establishment to establish the pumping-injection three-stage solute transport model.

In view of the situation that if a pollutant is put into the sample area, the water quality of the area can be destroyed, and a water source can be polluted. In some other possible embodiments, the pumping-injection three-stage solute transport model is trained on the basis of the sample water quality parameters of the aquifer in the sample area and second sample pumping-injection parameters collected for a water tracer injected into the sample area in the water injection stage, the standing stage and the water pumping stage of the aquifer. The tracer almost having no pollution to the water quality is selected to replace the pollutant, and the pumping-injection three-stage solute transport model is established by injecting the tracer into the sample area, and then, acquiring the second sample pumping-injection parameters collected for the tracer in the water injection stage, the standing stage and the water pumping stage of the aquifer. It should be noted that, at the moment, the second sample pumping-injection parameters include a starting time, a duration, a water injection speed and a water injection volume of the tracer in the water injection stage, the duration of the standing stage as well as the starting time, the duration and the water pumping speed of the water pumping stage.

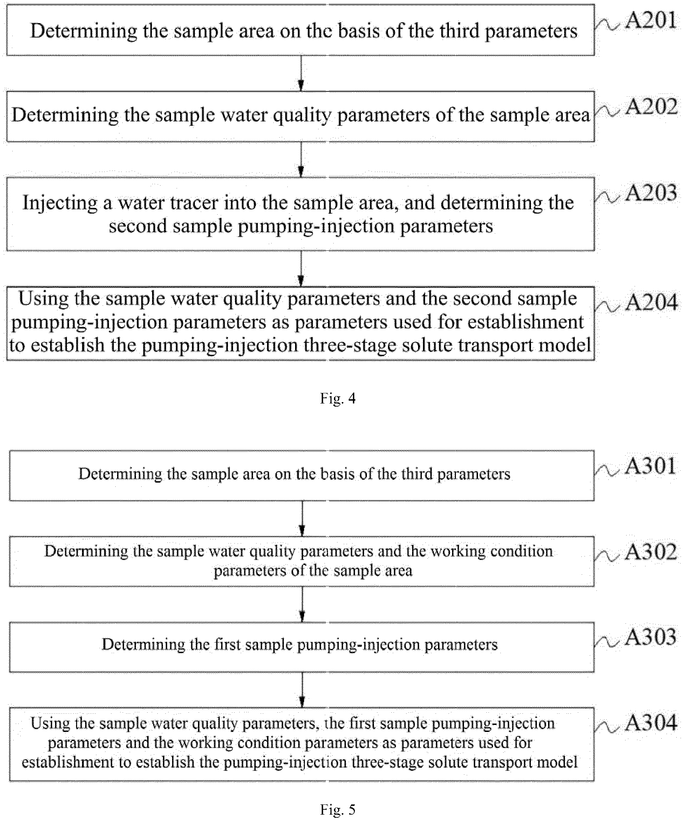

Accordingly, an example in which fluorescein is selected as the tracer is described. The groundwater pollutant control method based on single-well pumping-injection provided by the present disclosure will be described below in combination with Fig. 4. The pumping-injection three-stage solute transport model is established by the following steps.

A201, the sample area is determined on the basis of the third parameters.

In the present disclosure, when the sample area is screened, based on the third parameters which have been obtained and the known parameters of the underground medium structure as a candidate area, the candidate area similar to the underground medium structure in the to-be-controlled area is selected as the sample area. It can be understood that at least one candidate area can be selected as the sample area in the present disclosure.

A202, the sample water quality parameters of the sample area are determined. Similarly, the sample water quality parameters are the water quality parameters reflecting inhomogeneity and anisotropy.

A203, the water tracer is injected into the sample area; the concentration of the tracer in the water injection stage, the standing stage and the water pumping stage is continuously observed; experimental data is processed and collected; and the second sample pumping-injection parameters are determined.

A204, the sample water quality parameters and the second sample pumping-injection parameters are used as parameters used for establishment to establish the pumping-injection three-stage solute transport model.

In these embodiments, the pumping-injection three-stage solute transport model is a fractional derivative equation established under polar coordinates by stage-dividing on the basis of three stages including the water injection stage, the standing stage and the water pumping stage.

In the above-mentioned embodiment, the direction of the hydraulic gradient in the water injection stage and the water pumping stage may be changed, but a diffusion process is mainly taken into account in the standing stage due to a weak convection effect. The retention effect of a water-bearing medium on solute transport should be taken into account in all of the three stages. Therefore, it is necessary to establish a three-stage fractional-order convection-diffusion model, i.e., a pulse fractional-order model for three-stage solute transport under the polar coordinates to obtain the pumping-injection three-stage solute transport model. That is, for the water injection stage, the standing stage and the water pumping stage, the sample water quality parameters of the sample area as well as the first sample pumping-injection parameters/the second sample pumping-injection parameters are collected and are used as data for model establishment to establish the three-stage fractional-order convection-diffusion model applicable to the current solute transport process.

It should be noted that there is no standing stage under some conditions, that is, the duration of the standing stage is 0.

In the above-mentioned embodiment, the pumping-injection three-stage solute transport model is:

$$\begin{cases} \frac{\partial C(r,t)}{\partial t} + \beta_0 \,{}^{TC}_{\ 0}D_t^{\alpha_0,\lambda_0}C(r,t) = -\frac{v_0}{r}\frac{\partial C(r,t)}{\partial r} + \frac{D_0}{r}\frac{\partial^2 C(r,t)}{\partial r^2}, t \in [0,T_1] \\ \frac{\partial C(r,t)}{\partial t} + \beta_1 \,{}^{TC}_{\ T_1}D_t^{\alpha_1,\lambda_1}C(r,t) = \frac{D_1}{r}\frac{\partial^2 C(r,t)}{\partial r^2}, t \in [T_1,T_2] \\ \frac{\partial C(r,t)}{\partial t} + \beta_2 \,{}^{TC}_{\ T_2}D_t^{\alpha_2,\lambda_2}C(r,t) = -\frac{v_2}{r}\frac{\partial C(r,t)}{\partial r} + \frac{D_2}{r}\,{}^{RL}_{\ 0}D_r^{\gamma}C(r,t), t \in [T_2,T_3] \end{cases}$$

wherein r represents a transport radius of the groundwater pollutant; t represents a transport time of the groundwater pollutant; C represents a concentration of the groundwater pollutant; $[0, T_1]$ represents the duration of the water injection stage; $[T_1, T_2]$ represents the duration of the standing stage; $[T_2, T_3]$ represents the duration of the water pumping stage; $T_3$ represents the sum of the durations of the water injection stage, the standing stage and the water pumping stage; $\beta_0$ represents a fractional-order capacity coefficient of the water injection stage; $\beta_1$ represents a fractional-order capacity coefficient of the standing stage; $\beta_2$ represents a fractional-order capacity coefficient of the water pumping stage; $\lambda_0$ represents a truncation coefficient of the water injection stage; $\lambda_1$ represents a truncation coefficient of the standing stage; $\lambda_2$ represents a truncation coefficient of the water pumping stage; $\alpha_0$ represents a fractional order of the water injection stage; $\alpha_1$ represents a fractional order of the standing stage; $\alpha_2$ represents a temporal fractional order of the water pumping stage; $\gamma$ represents a spatial fractional order of the water pumping stage, $0 < \alpha_0, \alpha_1, \alpha_2 \leq 1$, $1 < \gamma \leq 2$, $\alpha_0, \alpha_1, \alpha_2$ and $\gamma$ are all determined on the basis of the inhomogeneity of the sample water quality parameters and are in negative correlation with the inhomogeneity of the sample water quality parameters as well as the inhomogeneity of each of the sample water quality parameters; $D_0$ represents a diffusion parameter of the water injection stage; $D_1$ represents a diffusion parameter of the standing stage; $D_2$ represents a diffusion parameter of the water pumping stage; $v_0$ represents a flow parameter of the water injection stage, $v_2$ represents a flow parameter of the water pumping stage, $v_0>0$, and $v_2<0$.

In the above-mentioned formula, fractional-order parameters are all dimensionless quantity and are applicable to the reflection of the inhomogeneity of an underground aquifer, i.e., the complexity of the underground medium. If the fractional order is low, the retention effect of the aquifer is strong, and it is possible that the fracture development is poor and there are more fatal weaknesses. Otherwise, the retention effect of the aquifer is weak. Moreover, the greater the difference of the fractional orders in the water injection stage and the water pumping stage is, the stronger the anisotropy of the aquifer is.

A specific value or value range of each parameter in the above-mentioned formula is calibrated on the basis of the sample water quality parameters which have been determined as well as the first sample pumping-injection parameters/the second sample pumping-injection parameters.

In some other possible embodiments, the tracer may be further selected from substances, such as sodium chloride (NaCl), existing in groundwater in the to-be-controlled area. Different from the above-mentioned embodiments, in these embodiments, actual working conditions including initial conditions and boundary conditions need to be taken into account, that is, the pumping-injection three-stage solute transport model is further trained on the basis of working condition parameters of the sample area, wherein the working condition parameters include an initial concentration and boundary conditions of the groundwater pollutant.

Accordingly, an example in which NaCl is selected as the tracer is described. The groundwater pollutant control method based on single-well pumping-injection provided by the present disclosure will be described below in combination with Fig. 5. The pumping-injection three-stage solute transport model is established by the following steps.

A301, the sample area is determined on the basis of the third parameters. This step is similar to step A101 and step A201, which is not described herein.

A302, the sample water quality parameters and the working condition parameters of the sample area are determined. Similarly, the sample water quality parameters and the working condition parameters may be determined on the basis of the single-well pumping-injection process.

A303, the first sample pumping-injection parameters are determined. In these embodiments, the first sample pumping-injection parameters include the starting time, the duration, the water injection speed and the water injection volume of the water injection stage, the duration of the standing stage as well as the starting time, the duration and the water pumping speed of the water pumping stage.

A304, the sample water quality parameters, the first sample pumping-injection parameters and the working condition parameters are used as parameters used for establishment to establish the pumping-injection three-stage solute transport model.

In these embodiments, the pumping-injection three-stage solute transport model is a fractional derivative equation established under polar coordinates by stage-dividing on the basis of three stages including the water injection stage, the standing stage and the water pumping stage.

In these embodiments, the pumping-injection three-stage solute transport model is:

$$
\begin{cases}
\frac{\partial C(r,t)}{\partial t} + \beta_0 \, {}^{TC}_0 D_t^{\alpha_0,\lambda_0} C(r,t) = -\frac{v_0}{r}\frac{\partial C(r,t)}{\partial r} + \frac{D_0}{r}\frac{\partial^2 C(r,t)}{\partial r^2}, t \in [0,T_1] \\
\frac{\partial C(r,t)}{\partial t} + \beta_1 \, {}^{TC}_{T_1} D_t^{\alpha_1,\lambda_1} C(r,t) = \frac{D_1}{r}\frac{\partial^2 C(r,t)}{\partial r^2}, t \in [T_1,T_2] \\
\frac{\partial C(r,t)}{\partial t} + \beta_2 \, {}^{TC}_{T_2} D_t^{\alpha_2,\lambda_2} C(r,t) = -\frac{v_2}{r}\frac{\partial C(r,t)}{\partial r} + \frac{D_2}{r} \, {}^{RL}_0 D_r^{\gamma} C(x,t), t \in [T_2,T_3] \\
\frac{\partial C(r,t)}{\partial r}\big|_{\partial\Omega} = 0 \\
C(r,0) = \begin{cases} A, r=0 \\ 0, else \end{cases}, C(r,T_1^+) = C(r,T_1^-), C(r,T_2^+) = C(r,T_2^-)
\end{cases}
$$

wherein r represents a transport radius of the groundwater pollutant; t represents a transport time of the groundwater pollutant; C represents a concentration of the groundwater pollutant; $[0,T_1]$ represents the duration of the water injection stage; $[T_1, T_2]$ represents the duration of the standing stage; $[T_2, T_3]$ represents the duration of the water pumping stage; $T_3$ represents the sum of the durations of the water injection stage, the standing stage and the water pumping stage; $\beta_0$ represents a fractional-order capacity coefficient of the water injection stage; $\beta_1$ represents a fractional-order capacity coefficient of the standing stage; $\beta_2$ represents a fractional-order capacity coefficient of the water pumping stage; $\lambda_0$ represents a truncation coefficient of the water injection stage; $\lambda_1$ represents a truncation coefficient of the standing stage; $\lambda_2$ represents a truncation coefficient of the water pumping stage; $\alpha_0$ represents a fractional order of the water injection stage; $\alpha_1$ represents a fractional order of the standing stage; $\alpha_2$ represents a temporal fractional order of the water pumping stage; y represents a spatial fractional order of the water pumping stage, $0 < \alpha_0, \alpha_1, \alpha_2 \leq 1, 1 < \gamma \leq 2, \alpha_0, \alpha_1, \alpha_2$ and $\gamma$ are all determined on the basis of the inhomogeneity of the sample water quality parameters and are in negative correlation with the inhomogeneity of the sample water quality parameters as well as the inhomogeneity of each of the sample water quality parameters; $D_0$ represents a diffusion parameter of the water injection stage; $D_1$ represents a diffusion parameter of the standing stage; $D_2$ represents a diffusion parameter of the water pumping stage; $v_0$ represents a flow parameter of the water injection stage, $v_2$ represents a flow parameter of the water pumping stage, $v_0 > 0$, $v_2 < 0$, A represents an initial concentration of the groundwater pollutant, and $\partial\Omega$ represents derivative calculation along a boundary.

A fractional derivative of truncation in a general form is defined as:

$$
{}^{TC}_{t_1} D_t^{\alpha,\lambda} C(r,t) = \frac{e^{-\lambda(t-t_1)}}{\Gamma(1-\alpha)} \int_{t_1}^{t} \frac{\left(e^{\lambda(\xi-t_1)} C(r,\xi)\right)'}{(t-\xi)^{\alpha}} d\xi
$$

A fractional derivative under the definition of Riemann-Liouville is defined as:

$$
{}^{RL}_0 D_r^{\gamma} C(x,r) = \frac{1}{\Gamma(2-\alpha)} \frac{d^2}{dr^2} \int_0^r (\zeta - r) C(\zeta,t) d\zeta
$$

wherein $\xi$ represents a functional integration of t, $\zeta$ represents a functional integration of a space x, $t \in [t1, t2]$, t1 represents a memory starting time of the fractional derivative and is generally an initial time of each stage, t2 represents a memory ending time of the fractional derivative and is generally an ending time of each stage, e represents a natural constant, $\Gamma$ represents a Gamma function of single parameter and is defined as follows:

$$\Gamma(\gamma) = \int_0^{+\infty} s^{\gamma-1} e^{-s}\, ds$$

wherein $\gamma$ represents a single-parameter variable in the gamma function.

In the present disclosure, the pumping-injection three-stage solute transport model is solved by using an implicit finite difference method. Specifically,

when $t \in [0, T_1]$, the number of points in time is $m$, the time step is $\tau = T_1/m$, $t_{k+1}$ represents the $(k+1)^{\text{th}}$ time, and $r_l$ represents the $l^{\text{th}}$ spatial position. At the moment,

when k=0,

$$\left(\tau^{-1} + \beta_0 \frac{\tau^{-\alpha_0}}{\Gamma(2-\alpha_0)} - \frac{2D_0}{r_l h^2}\right) C(r_l, t_1) + \left(\frac{v_0}{r_l h} + \frac{D_0}{r_l h^2}\right) C(r_{l+1}, t_1)$$

$$+ \left(-\frac{v_0}{h r_l} + \frac{D_0}{r_l h^2}\right) C(r_{l-1}, t_1) = \tau^{-1} C(r_l, 0) - \left(\beta_0 \frac{e^{-\lambda_0 t_1} \tau^{-\alpha_0}}{\Gamma(2-\alpha_0)}\right) C(r_l, 0)$$

when k=1,

$$\left(\tau^{-1} + \beta_0 \frac{\tau^{-\alpha_0}}{\Gamma(2-\alpha_0)} - \frac{2D_0}{r_l h^2}\right) C(r_l, t_2) + \left(\frac{v_0}{r_l h} + \frac{D_0}{r_l h^2}\right) C(r_{l+1}, t_2)$$

$$+ \left(-\frac{v_0}{h r_l} + \frac{D_0}{r_l h^2}\right) C(r_{l-1}, t_2) = \tau^{-1} C(r_l, t_1) - \left(\beta_0 \frac{e^{-\lambda_0 t_2} \tau^{-\alpha_0}}{\Gamma(2-\alpha_0)}\right) \left[\begin{array}{c} e^{\lambda_0 t_1} C(r_l, t_1)(2^{1-\alpha_0} - 2) \\ -C(r_l, 0)(2^{1-\alpha_0} - 1) \end{array}\right]$$

when $k \geq 1$,

$$\left(\tau^{-1} + \beta_0 \frac{\tau^{-\alpha_0}}{\Gamma(2-\alpha_0)} - \frac{2D_0}{r_l h^2}\right) C(r_l, t_{k+1}) + \left(\frac{v_0}{r_l h} + \frac{D_0}{r_l h^2}\right) C(r_{l+1}, t_{k+1})$$

$$+ \left(-\frac{v_0}{r_l h} + \frac{D_0}{r_l h^2}\right) C(r_{l-1}, t_{k+1}) = \tau^{-1} C(r_l, t_k) - \beta_0 \frac{e^{-\lambda_0 t_{k+1}} \tau^{-\alpha_0}}{\Gamma(2-\alpha_0)} e^{\lambda_0 t_k} C(r_l, t_k)(2^{1-\alpha_0} - 2)$$

$$- \beta_0 \frac{e^{-\lambda_0 t_{k+1}} \tau^{-\alpha_0}}{\Gamma(2-\alpha_0)} \sum_{j=1}^{k-1} e^{-\lambda_0 t_{k-j}} C(r_l, t_{k-j})[(j+2)^{1-\alpha_0} - 2(j+1)^{1-\alpha_0} + j^{1-\alpha_0}]$$

$$+ \beta_0 \frac{e^{-\lambda_0 t_{k+1}} \tau^{-\alpha_0}}{\Gamma(2-\alpha_0)} C(r_l, 0)\left[j_{k+1}^{1-\alpha_0} - j_k^{1-\alpha_0}\right].$$

When $t \in [T_1, T_2]$, the number of points in time is $m1$, the time step is $\tau_1 = (T_2 - T_1)/m1$, $t_{k+1}$ represents the $(k+1)^{\text{th}}$ time, and $r_l$ represents the $l^{\text{th}}$ spatial position. At the moment,

when $k - m = 0$,

$$\left(\tau_1^{-1} + \beta_1 \frac{\tau_1^{-\alpha_1}}{\Gamma(2-\alpha_1)} - \frac{2D_1}{r_l h^2}\right) C(r_l, t_{k+1}) + \frac{D_1}{r_l h^2} C(r_{l+1}, t_{k+1})$$

$$+ \frac{D_1}{r_l h^2} C(r_{l-1}, t_{k+1}) = \tau_1^{-1} C(r_l, t_k) - \left(\beta_1 \frac{e^{-\lambda_1 (t_{k+1} - T_1)} \tau_1^{-\alpha_1}}{\Gamma(2-\alpha_1)}\right) C(r_l, T_1)$$

when $k - m = 1$,

**[00125]**
$$\left(\tau_1^{-1} + \beta_1 \frac{\tau_1^{-\alpha_1}}{\Gamma(2-\alpha_1)} - \frac{2D_1}{r_l h^2}\right) C(r_l, t_{k+1}) + \frac{D_1}{r_l h^2} C(r_{l+1}, t_{k+1})$$

$$+ \frac{D_1}{r_l h^2} C(r_{l-1}, t_{k+1}) = \tau_1^{-1} C(r_l, t_k) - \left(\beta_1 \frac{e^{-\lambda_1(t_{k+1}-T_1)}\tau_1^{-\alpha_1}}{\Gamma(2-\alpha_1)}\right)\begin{bmatrix} e^{\lambda_1(t_k-T_1)} C(r_l, t_k)(2^{1-\alpha_1}-2) \\ -C(r_l, T_1)(2^{1-\alpha_1}-1) \end{bmatrix}$$

when $k - m \geq 1$,

$$\left(\tau_1^{-1} + \beta_1 \frac{\tau_1^{-\alpha_1}}{\Gamma(2-\alpha_1)} - \frac{2D_1}{r_l h^2}\right) C(r_l, t_{k+1}) + \frac{D_1}{r_l h^2} C(r_{l+1}, t_{k+1})$$

$$+ \frac{D_1}{r_l h^2} C(r_{l-1}, t_{k+1}) = \tau_1^{-1} C(r_l, t_k) - \beta_1 \frac{e^{-\lambda_1(t_{k+1}-T_1)}\tau_1^{-\alpha_1}}{\Gamma(2-\alpha_1)} e^{\lambda_1(t_{k+1}-T_1)} C(r_l, t_k)(2^{1-\alpha_1}-2)$$

$$- \beta_1 \frac{e^{-\lambda_1(t_{k+1}-T_1)}\tau_1^{-\alpha_1}}{\Gamma(2-\alpha_1)} \sum_{j=1}^{k-m-1} e^{-\lambda_1(t_{k-j}-T_1)} C(r_l, t_{k-j})[(j+2)^{1-\alpha_1} - 2(j+1)^{1-\alpha_1} + j^{1-\alpha_1}]$$

$$+ \beta_1 \frac{e^{-\lambda_1(t_{k+1}-T_1)}\tau_1^{-\alpha_1}}{\Gamma(2-\alpha_1)} C(r_l, T_1)[j_{k+1}^{1-\alpha_1} - j_k^{1-\alpha_1}].$$

When $t \in [T_2, T_3]$, the number of points in time is $m2$, the time step is $\tau_2 = (T_3 - T_2)/m2$, $t_{k+1}$ represents the $(k+1)^{th}$ time, $r_l$ represents the $l^{th}$ spatial position, the spatial length is $L$, the number of points in space is $n=L/h+1$, and $g_0 = 1$, $g_i = -\frac{\gamma-i+1}{i} g_{i-1}$, and $i = 1,2, \ldots, n$. At the moment,

when $k - m - m1 = 0$,

$$\left(\tau_2^{-1} + \beta_2 \frac{\tau_2^{-\alpha_2}}{\Gamma(2-\alpha_2)} - \frac{g_1 D_2}{r_l h^\gamma}\right) C(r_l, t_{k+1}) + \left(\frac{v_2}{r_l h} + \frac{g_0 D_2}{r_l h^\gamma}\right) C(r_{l+1}, t_{k+1})$$

$$+ \frac{D_2}{r_l h^\gamma} \sum_{i=2}^{l+1} g_i C(r_{l-i+1}, t_{k+1}) - \frac{v_2}{r_l h} C(r_{l-1}, t_{k+1}) = \tau_2^{-1} C(r_l, T_2) - \left(\beta_2 \frac{e^{-\lambda_2(t_{k+1}-T_2)}\tau_2^{-\alpha_2}}{\Gamma(2-\alpha_2)}\right) C(r_l, T_2)$$

when $k - m - m1 = 1$,

$$\left(\tau_2^{-1} + \beta_2 \frac{\tau_2^{-\alpha_2}}{\Gamma(2-\alpha_2)} - \frac{2D_2}{r_l h^\gamma}\right) C(r_l, t_{k+1}) + \left(\frac{v_2}{r_l h} + \frac{D_2}{r_l h^\gamma}\right) C(r_{l+1}, t_{k+1})$$

$$+ \frac{D_2}{r_l h^\gamma} \sum_{i=2}^{l+1} g_i C(r_{l-i+1}, t_{k+1}) - \frac{v_2}{r_l h} C(r_{l-1}, t_{k+1}) = \tau_2^{-1} C(r_l, t_k)$$

$$- \left(\beta_2 \frac{e^{-\lambda_2(t_{k+1}-T_2)}\tau_2^{-\alpha_2}}{\Gamma(2-\alpha_2)}\right)\begin{bmatrix} e^{\lambda_2(t_k-T_2)} C(r_l, t_k)(2^{1-\alpha_2}-2) \\ -C(r_l, T_2)(2^{1-\alpha_2}-1) \end{bmatrix}$$

when $k - m - m1 \geq 1$,

$$\left(\tau_2^{-1} + \beta_2 \frac{\tau_2^{-\alpha_2}}{\Gamma(2-\alpha_2)} - \frac{2D_2}{r_l h^\gamma}\right) C(r_l, t_{k+1}) + \left(\frac{v_2}{r_l h} + \frac{D_2}{r_l h^\gamma}\right) C(r_{l+1}, t_{k+1})$$

$$+\frac{D_2}{r_l h^\gamma} \sum_{i=2}^{l+1} g_i C(r_{l-i+1}, t_{k+1}) - \frac{v_2}{r_l h} C(r_{l-1}, t_{k+1}) = \tau_2^{-1} C(r_l, t_k)$$

$$-\beta_2 \frac{e^{-\lambda_2(t_{k+1}-T_2)} \tau_2^{-\alpha_2}}{\Gamma(2-\alpha_2)} e^{\lambda_2(t_{k+1}-T_2)} C(r_l, t_k)(2^{1-\alpha_2} - 2)$$

$$-\beta_2 \frac{e^{-\lambda_2(t_{k+1}-T_2)} \tau_2^{-\alpha_2}}{\Gamma(2-\alpha_2)} \sum_{j=1}^{k-m-m1-1} e^{-\lambda_2(t_{k-j}-T_2)} C(r_l, t_{k-j})[(j+2)^{1-\alpha_2} - 2(j+1)^{1-\alpha_2} + j^{1-\alpha_2}]$$

$$+\beta_2 \frac{e^{-\lambda_2(t_{k+1}-T_2)} \tau_2^{-\alpha_2}}{\Gamma(2-\alpha_2)} C(r_l, T_2)\left[j_{k+1}^{1-\alpha_2} - j_k^{1-\alpha_2}\right].$$

A weathered fractured granite geology in the to-be-controlled area will be described as an example. According to the solution of the present disclosure, a control plan for a groundwater pollutant in an area having the weathered fractured granite geology may be drawn up.

Fracture development of a crystalline rock in the granite geology is very poor, and there are few long communicating cracks. Therefore, due to the transient change of the size and direction of the hydraulic gradient occurring in the three different hydraulic stages including the water injection stage, the standing stage and the water pumping stage, an over-diffusion process may occur in a solute direction in the water pumping stage. In order to protect the geological environment of a weathered fractured granite, firstly, geological drilling may be performed in the geological environment, and a single-well tracer experiment as well as corresponding value simulation and recording during the experiment required in the present disclosure are performed on a drilling position. It can be understood that a plurality of observation wells may be selected in the present disclosure. Specifically, in the present disclosure, two CH3 and CH10 observation wells are selected for the single-well tracer experiment, and NaCl is selected as the tracer.

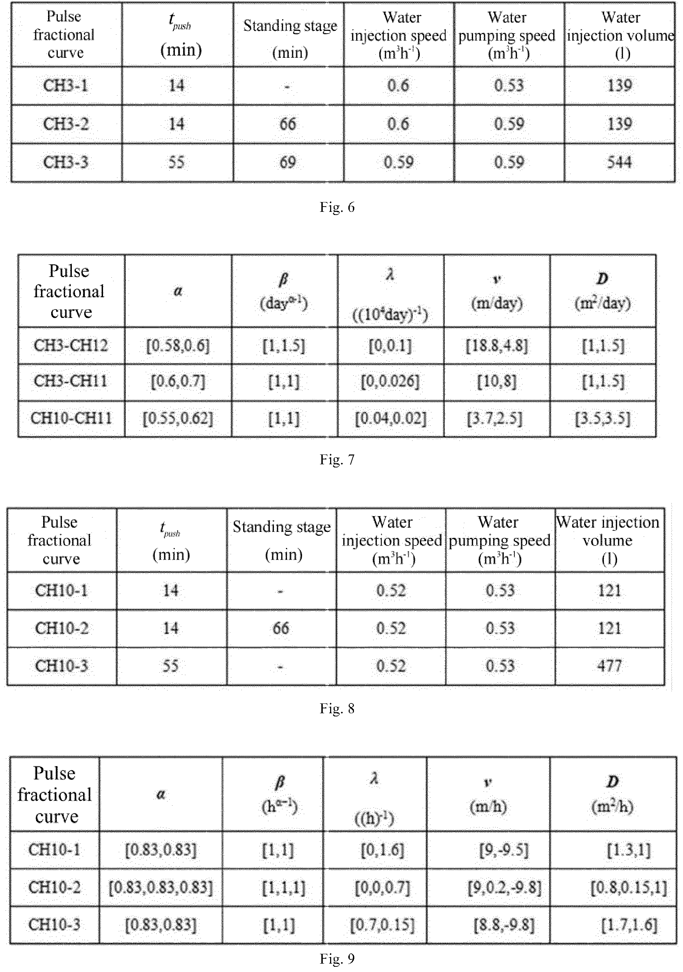

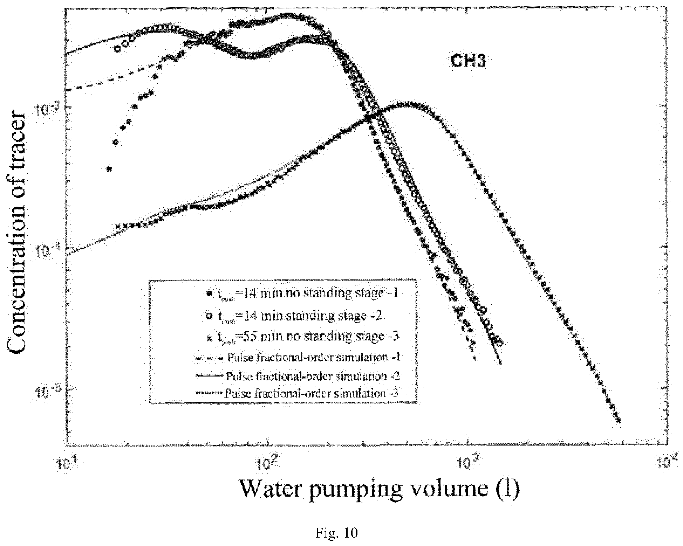

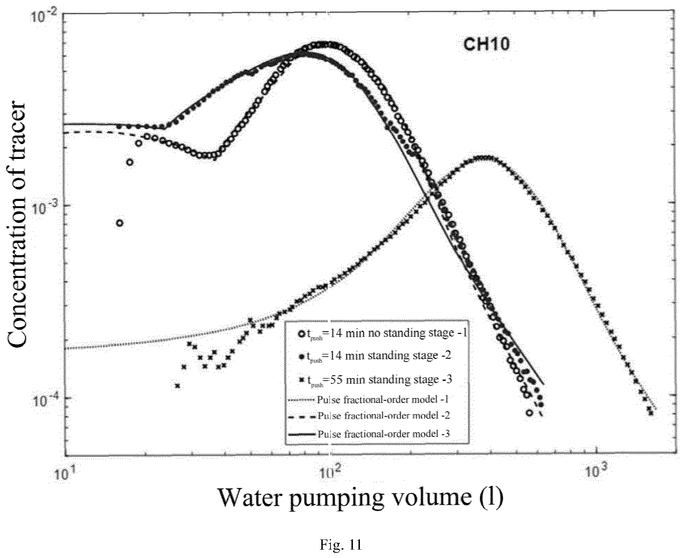

At the moment, experimental conditions of the two CH3 and CH10 observation wells are respectively shown in Fig. 6 and Fig. 8, and model simulation results obtained after experimental data is collected are respectively shown in Fig. 10 and Fig. 11.

It can be seen from Fig. 10 and Fig. 11 that the pumping-injection three-stage solute transport model in the present disclosure can favorably depict a process of peak arrival and gradual attenuation of sub-diffusion of tracer particles. When the water injection time is shorter and there is no standing stage, the fractional truncation coefficient of the model is higher, which shows that the retention process of the solute transport is more rapidly attenuated; when the injection time of the tracer particles is short and there is no standing stage, part of the tracer may be stored in a non-communicating fracture closer to the wells, and during water pumping, the particles rapidly flow back to a test well along a channel of the fracture, which is shown as a phenomenon of early peak on a breakthrough curve; and when the injection time of the tracer is longer and there is the standing stage, the phenomenon of early peak cannot be observed, and a phenomenon of early arrival in a solute backflow process is nonobvious. At the moment, the truncation coefficient of the model is higher, which is related to the fact that the particles find a flowback channel easily during water pumping. Therefore, it can be inferred that the pollutant solute is more easily cleaned in the water pumping stage of the single well in this area.

A groundwater pollutant control apparatus based on single-well pumping-injection provided by the present disclosure will be described below, and the groundwater pollutant control apparatus based on single-well pumping-injection described hereinafter and the groundwater pollutant control method based on single-well pumping-injection described above may correspondingly refer to each other.

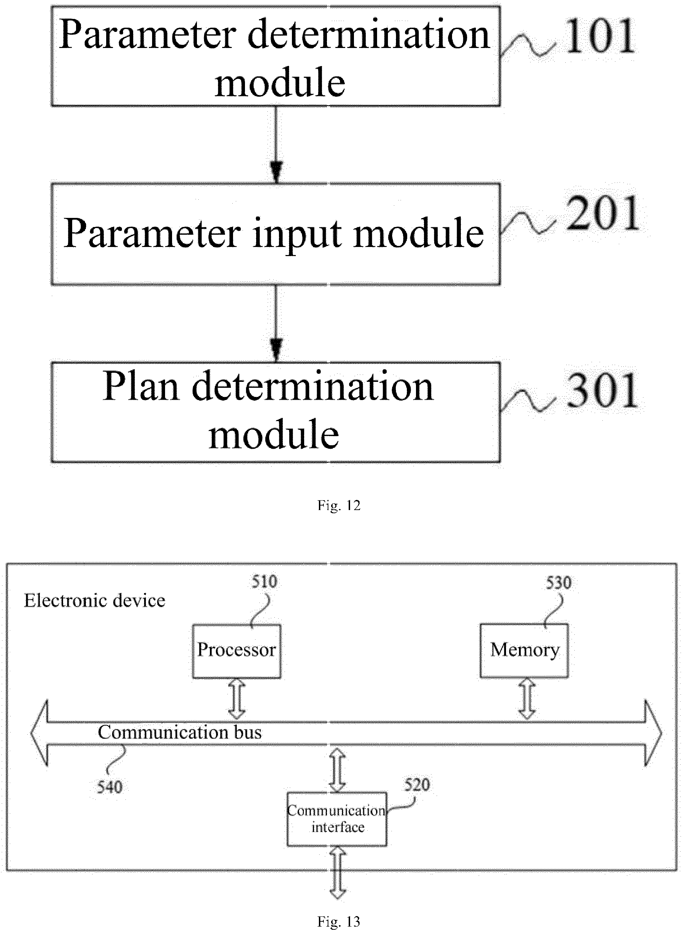

The groundwater pollutant control apparatus based on single-well pumping-injection in the present disclosure will be described below in combination with Fig. 12, and the present disclosure is implemented on the basis of a single-well pumping-injection process. The apparatus includes

a parameter determination module 101 configured to determine first parameters, second parameters and third parameters of a to-be-controlled area; wherein

in the present embodiment, the first parameters characterize parameters of a groundwater pollutant in the to-be-controlled area in a water injection stage, the second parameters characterize parameters of the groundwater pollutant in a standing stage, and the third parameters characterize hydraulic parameters of an aquifer in the to-be-controlled area; and

a single-well pumping-injection process is mainly divided into three stages, i.e., a water injection stage, a standing stage and a water pumping stage, and the water injection stage, the standing stage and the water pumping stage are performed in sequence, that is, the standing stage begins after the water injection stage ends, and the water pumping stage begins after the standing stage ends;

a parameter input module 102 configured to input the first parameters, the second parameters and the third parameters into a pumping-injection three-stage solute transport model to obtain fourth parameters output by the pumping-injection three-stage solute transport model; and

a plan determination module 103 configured to draw up a control plan for the to-be-controlled area on the basis of the fourth parameters, wherein by using the control plan, corresponding control on groundwater pollution is performed.

According to the groundwater pollutant control apparatus based on single-well pumping-injection provided by the present disclosure, based on the first two physical stages of the single-well pumping-injection and geological characteristics of the to-be-controlled area, and particularly based on the hydraulic parameters, capable of reflecting the inhomogeneity and anisotropy of an underground medium in the to-be-controlled area to a certain extent, of the aquifer in the to-be-controlled area, a transport process of the groundwater pollutant, namely a groundwater solute, is simulated by means of the pumping-injection three-stage solute transport model to know about a distribution situation of the groundwater pollutant in the to-be-controlled area, based on which the pollution level of groundwater is evaluated. Various parameters of the groundwater pollutant in the third stage of the single-well pumping-injection are predicted according to the distribution situation predicted by the pumping-injection three-stage solute transport model, and then, specific parameters (indexes) required for physical cleaning in the to-be-controlled area are set, so that the control plan is more applicable to a physical structure of the underground medium in the to-be-controlled area, and can better evaluate and remediate groundwater pollution, be beneficial to the application of groundwater dynamics in production and life, and provide corresponding support for evaluation and remediation of the groundwater pollution.

Fig. 13 illustrates a schematic structural diagram of an entity of an electronic device. As shown in Fig. 13, the electronic device may include a processor 510, a communication interface 520, a memory 530, and a communication bus 540, wherein communication among the processor 510, the communication interface 520 and the memory 530 is completed via the communication bus 540. The processor 510 may call a logic command in the memory 530 so as to perform the groundwater pollutant control method based on single-well pumping-injection. The method includes the following steps:

first parameters, second parameters and third parameters of a to-be-controlled area are determined; the first parameters characterize parameters of a groundwater pollutant in the to-be-controlled area in a water injection stage; the second parameters characterize parameters of the groundwater pollutant in a standing stage; and the third parameters characterize hydraulic parameters of an aquifer in the to-be-controlled area;

the first parameters, the second parameters and the third parameters are input into a pumping-injection three-stage solute transport model to obtain fourth parameters output by the pumping-injection three-stage solute transport model; the pumping-injection three-stage solute transport model is trained on the basis of sample water quality parameters of an aquifer in a sample area and first sample pumping-injection parameters collected for a groundwater pollutant in the sample area in the water injection stage, the standing stage and a water pumping stage, and the water injection stage, the standing stage and the water pumping stage are performed in sequence; the fourth parameters characterize parameters of the groundwater pollutant in the water pumping stage and include a starting time, a duration and a water pumping speed of the water pumping stage; and

a control plan for the to-be-controlled area is drawn up on the basis of the fourth parameters.

In addition, the above-mentioned logic command in the memory 530 may be implemented in a form of a software functional unit and may be stored in a computer-readable storage medium when sold or used as an independent product. Based on such understanding, the technical solutions of the present disclosure in essence or parts making contributions to the prior art or part of the technical solutions may be embodied in a form of a software product. The computer software product is stored in a storage medium, and includes a plurality of commands used to enable a computer device (which may be a personal computer, a server, or a network device, etc.,) to perform all or part of the steps of the method in each of the embodiments of the present disclosure. The aforementioned storage medium includes various media capable of storing program codes, such as a U disk, a mobile hard disk, a read-only memory (ROM), a random access memory (RAM), a diskette or an optical disk,.

On the other hand, the present disclosure further provides a computer program product. The computer program product includes a computer program, and the computer program may be stored in a non-transitory computer-readable storage medium. When the computer program is executed by a processor, a computer may perform the groundwater pollutant control method based on single-well pumping-injection provided in each of the above-mentioned embodiments. The method includes the following steps:

first parameters, second parameters and third parameters of a to-be-controlled area are determined; the first parameters characterize parameters of a groundwater pollutant in the to-be-controlled area in a water injection stage; the second parameters characterize parameters of the groundwater pollutant in a standing stage; the third parameters characterize hydraulic parameters of an aquifer in the to-be-controlled area;

the first parameters, the second parameters and the third parameters are input into a pumping-injection three-stage solute transport model to obtain fourth parameters output by the pumping-injection three-stage solute transport model; the pumping-injection three-stage solute transport model is trained on the basis of sample water quality parameters of an aquifer in a sample area and first sample pumping-injection parameters collected for a groundwater pollutant in the sample area in the water injection stage, the standing stage and a water pumping stage, and the water injection stage, the standing stage and the water pumping stage are performed in sequence; and the fourth parameters characterize parameters of the groundwater pollutant in the water pumping stage and include a starting time, a duration and a water pumping speed of the water pumping stage; and

a control plan for the to-be-controlled area is drawn up on the basis of the fourth parameters.

In further aspect, the present disclosure further provides a non-transitory computer-readable storage medium, with a computer program stored therein, wherein the computer program, when executed by a processor, implements the groundwater pollutant control method based on single-well pumping-injection provided in each of the above-mentioned embodiments. The method includes the following steps:

first parameters, second parameters and third parameters of a to-be-controlled area are determined; the first parameters characterize parameters of a groundwater pollutant in the to-be-controlled area in a water injection stage; the second parameters characterize parameters of the groundwater pollutant in a standing stage; and the third parameters characterize hydraulic parameters of an aquifer in the to-be-controlled area;

the first parameters, the second parameters and the third parameters are input into a pumping-injection three-stage solute transport model to obtain fourth parameters output by the pumping-injection three-stage solute transport model; the pumping-injection three-stage solute transport model is trained on the basis of sample water quality parameters of an aquifer in a sample area and first sample pumping-injection parameters collected for a groundwater pollutant in the sample area in the water injection stage, the standing stage and a water pumping stage, and the water injection stage, the standing stage and the water pumping stage are performed in sequence; and the fourth parameters characterize parameters of the groundwater pollutant in the water pumping stage and include a starting time, a duration and a water pumping speed of the water pumping stage; and

a control plan for the to-be-controlled area is drawn up on the basis of the fourth parameters.

The apparatus embodiment described above is merely illustrative, wherein the units described as separated components may be or not be physically separated, and components serving as units for displaying may be or not be physical units, that is, may be located on the same place or distributed on a plurality of network units. Part or all of the modules may be selected according to an actual demand to achieve the objectives of the solution in the present embodiment. The present disclosure may be understood and implemented by those of ordinary skill in the art without creative work.

Through the description for the above-mentioned implementations, those skilled in the art may clearly know that all the implementations may be implemented by means of software and an essential universal hardware platform, and certainly may also be implemented by means of hardware. Based on such understanding, the above-mentioned technical solutions in essence or parts making contributions to the prior art may be embodied in a form of a software product, and the computer software product may be stored in a computer-readable storage medium such as a ROM/RAM, a diskette, and an optical disk, and includes a plurality of commands used to enable a computer device (which may be a personal computer, a server, or a network device, etc.,) to perform the method in each of the embodiments or some parts of the embodiments.

Finally, it should be noted that the above-mentioned embodiments are merely intended to describe the technical solutions of the present disclosure, rather than to limit the present disclosure. Although the present disclosure has been described in detail with reference to the aforementioned embodiments, it should be understood by those of ordinary skill in the art that the technical solutions recorded in each of the foregoing embodiments may still be modified under the scope of appended claims.

**Claims**

1.  A computer-implemented groundwater pollutant control method by a single-well pumping-injection process, the method comprises:

    determining first parameters, second parameters and third parameters of a to-be-controlled area; the first parameters characterizing parameters of a groundwater pollutant in a to-be-controlled area in a a water injection stage, the second parameters characterizing parameters of the groundwater pollutant in a standing stage; and the third parameters characterizing hydraulic parameters of an aquifer in the to-be-controlled area and comprising water quality parameters reflecting inhomogeneity and anisotropy; inputting the first parameters, the second parameters and the third parameters into a pumping-injection three-stage solute transport model to obtain fourth parameters output by the pumping-injection three-stage solute transport model; the pumping-injection three-stage solute transport model being trained on the basis of sample water quality parameters of an aquifer in a sample area and first sample pumping-injection parameters collected for a groundwater pollutant in the sample area in the water injection stage, the standing stage and a water pumping stage, and the water injection stage, the standing stage and the water pumping stage being performed in sequence; and the fourth parameters characterizing parameters of the groundwater pollutant in the water pumping stage and comprising a starting time, a duration and a water pumping speed of the water pumping stage; and drawing up a control plan for the to-be-controlled area on the basis of the fourth parameters; wherein the pumping-injection three-stage solute transport model is established by the following steps:

    determining the sample area on the basis of the third parameters; determining the sample water quality parameters of the sample area; the sample water quality parameters being the water quality parameters reflecting inhomogeneity and anisotropy; determining the first sample pumping-injection parameters; the first sample pumping-injection parameters comprising a starting time, a duration, a water injection speed and a water injection volume of the groundwater pollutant in the water injection stage, a duration of the standing stage as well as the starting time, the duration and the water pumping speed of the water pumping stage; using the sample water quality parameters and the first sample pumping-injection parameters as parameters used for establishment to establish the pumping-injection three-stage solute transport model; the pumping-injection three-stage solute transport model being a fractional derivative equation established under polar coordinates by stage-dividing on the basis of three stages comprising the water injection stage, the standing stage and the water pumping stage; wherein the pumping-injection three-stage solute transport model is:

$$
\begin{cases}
\dfrac{\partial C(r,t)}{\partial t} + \beta_0\, {}^{TC}_{0}D^{\alpha_0,\lambda_0}_t C(r,t) = -\dfrac{v_0}{r}\dfrac{\partial C(r,t)}{\partial r} + \dfrac{D_0}{r}\dfrac{\partial^2 C(r,t)}{\partial r^2}, t \in [0,T_1] \\[2ex]
\dfrac{\partial C(r,t)}{\partial t} + \beta_1\, {}^{TC}_{T_1}D^{\alpha_1,\lambda_1}_t C(r,t) = \dfrac{D_1}{r}\dfrac{\partial^2 C(r,t)}{\partial r^2}, t \in [T_1,T_2] \\[2ex]
\dfrac{\partial C(r,t)}{\partial t} + \beta_2\, {}^{TC}_{T_2}D^{\alpha_2,\lambda_2}_t C(r,t) = -\dfrac{v_2}{r}\dfrac{\partial C(r,t)}{\partial r} + \dfrac{D_2}{r}\, {}^{4L}_{0}D^{\gamma}_r C(r,t), t \in [T_2,T_3]
\end{cases}
$$

    wherein r represents a transport radius of the groundwater pollutant; t represents a transport time of the groundwater pollutant; C represents a concentration of the groundwater pollutant; $[0,T_1]$ represents the duration of the water injection stage; $[T_1,T_2]$ represents the duration of the standing stage; $[T_2,T_3]$ represents the duration of the water pumping stage; $T_3$ represents the sum of the durations of the water injection stage, the standing stage and the water pumping stage; $\beta_0$ represents a fractional-order capacity coefficient of the water injection stage; $\beta_1$: represents a fractional-order capacity coefficient of the standing stage; $\beta_2$ represents a fractional-order capacity coefficient of the water pumping stage; $\lambda_0$ represents a truncation

coefficient of the water injection stage; $\lambda_1$ represents a truncation coefficient of the standing stage; $\lambda_2$ represents a truncation coefficient of the water pumping stage; $\alpha_0$ represents a fractional order of the water injection stage; $\alpha_1$ represents a fractional order of the standing stage; $\alpha_2$ represents a temporal fractional order of the water pumping stage; $\gamma$ represents a spatial fractional order of the water pumping stage, $0 < \alpha_0$, $\alpha_1, \alpha_2 \leq 1$, $1 < \gamma \leq 2$, $\alpha_0, \alpha_1, \alpha_2$ and $\gamma$ are all determined on the basis of the inhomogeneity of the sample water quality parameters and are in negative correlation with the inhomogeneity of the sample water quality parameters as well as the inhomogeneity of each of the sample water quality parameters; $D_0$ represents a diffusion parameter of the water injection stage; $D_1$ represents a diffusion parameter of the standing stage; $D_2$ represents a diffusion parameter of the water pumping stage; $v_0$ represents a flow parameter of the water injection stage, $v_2$ represents a flow parameter of the water pumping stage, $v_0>0$, and $v_2<0$.

2. The method of claim 1, **characterized in that** the step of determining the first parameters, the second parameters and the third parameters of a to-be-controlled area comprises:

determining the third parameters on the basis of exploration data of the to-be-controlled area; the third parameters comprising water quality parameters reflecting inhomogeneity and anisotropy; and
determining the first parameters and the second parameters; the first parameters comprising the starting time, the duration, the water injection speed and the water injection volume of the water injection stage; and the second parameters being the duration of the standing stage.

3. The method of claim 1, **characterized in that** the pumping-injection three-stage solute transport model is trained on the basis of the sample water quality parameters of the aquifer in the sample area and second sample pumping-injection parameters collected for a water tracer injected into the sample area in the water injection stage, the standing stage and the water pumping stage of the aquifer;
accordingly, the pumping-injection three-stage solute transport model is established by further comprising the following steps:

injecting the water tracer into the sample area, and determining the second sample pumping-injection parameters; the second sample pumping-injection parameters comprising a starting time, a duration, a water injection speed and a water injection volume of the tracer in the water injection stage, a duration of the standing stage as well as a starting time, a duration and a water pumping speed of the water pumping stage; and
using the sample water quality parameters and the second sample pumping-injection parameters as parameters used for establishment to establish the pumping-injection three-stage solute transport model; the pumping-injection three-stage solute transport model being a fractional derivative equation established under polar coordinates by stage-dividing on the basis of three stages comprising the water injection stage, the standing stage and the water pumping stage.

4. The method of claim 3, **characterized in that** the pumping-injection three-stage solute transport model is further trained on the basis of working condition parameters of the sample area; the working condition parameters comprise an initial concentration and boundary conditions of the groundwater pollutant;
accordingly, the pumping-injection three-stage solute transport model is established by the following steps:

determining the sample area on the basis of the third parameters;
determining the sample water quality parameters and the working condition parameters of the sample area;
determining the first sample pumping-injection parameters; the first sample pumping-injection parameters comprising the starting time, the duration, the water injection speed and the water injection volume of the water injection stage, the duration of the standing stage as well as the starting time, the duration and the water pumping speed of the water pumping stage; and
using the sample water quality parameters, the first sample pumping-injection parameters and the working condition parameters as parameters used for establishment to establish the pumping-injection three-stage solute transport model; the pumping-injection three-stage solute transport model being a fractional derivative equation established under polar coordinates by stage-dividing on the basis of three stages comprising the water injection stage, the standing stage and the water pumping stage.

5. The method of claim 4, **characterized in that** the pumping-injection three-stage solute transport model is:

$$\begin{cases} \frac{\partial C(r,t)}{\partial t} + \beta_0 \, {}^{TC}_0 D_t^{\alpha_0,\lambda_0} C(r,t) = -\frac{v_0}{r}\frac{\partial C(r,t)}{\partial r} + \frac{D_0}{r}\frac{\partial^2 C(r,t)}{\partial r^2}, t \in [0,T_1] \\ \frac{\partial C(r,t)}{\partial t} + \beta_1 \, {}^{TC}_{T_1} D_t^{\alpha_1,\lambda_1} C(r,t) = \frac{D_1}{r}\frac{\partial^2 C(r,t)}{\partial r^2}, t \in [T_1,T_2] \\ \frac{\partial C(r,t)}{\partial t} + \beta_2 \, {}^{TC}_{T_2} D_t^{\alpha_2,\lambda_2} C(r,t) = -\frac{v_2}{r}\frac{\partial C(r,t)}{\partial r} + \frac{D_2}{r} \, {}^{RL}_0 D_r^{\gamma} C(x,t), t \in [T_2,T_3] \\ \frac{\partial C(r,t)}{\partial r}\big|_{\partial\Omega} = 0 \\ C(r,0) = \begin{cases} A, r = 0 \\ 0, else \end{cases}, C(r,T_1^+) = C(r,T_1^-), C(r,T_2^+) = C(r,T_2^-) \end{cases}$$

wherein r represents a transport radius of the groundwater pollutant; t represents a transport time of the groundwater pollutant; C represents a concentration of the groundwater pollutant; $[0,T_1]$ represents the duration of the water injection stage; $[T_1,T_2]$ represents the duration of the standing stage; $[T_2,T_3]$ represents the duration of the water pumping stage; $T_3$ represents the sum of the durations of the water injection stage, the standing stage and the water pumping stage; $\beta_0$ represents a fractional-order capacity coefficient of the water injection stage; $\beta_1$: represents a fractional-order capacity coefficient of the standing stage; $\beta_2$ represents a fractional-order capacity coefficient of the water pumping stage; $\lambda_0$ represents a truncation coefficient of the water injection stage; $\lambda_1$ represents a truncation coefficient of the standing stage; $\lambda_2$ represents a truncation coefficient of the water pumping stage; $\alpha_0$ represents a fractional order of the water injection stage; $\alpha_1$ represents a fractional order of the standing stage; $\alpha_2$ represents a temporal fractional order of the water pumping stage; $\gamma$ represents a spatial fractional order of the water pumping stage, $0 < \alpha_0, \alpha_1, \alpha_2 \leq 1$, $1 < \gamma \leq 2$, $\alpha_0, \alpha_1, \alpha_2$ and $\gamma$ are all determined on the basis of the inhomogeneity of the sample water quality parameters and are in negative correlation with the inhomogeneity of the sample water quality parameters as well as the inhomogeneity of each of the sample water quality parameters; $D_0$ represents a diffusion parameter of the water injection stage; $D_1$ represents a diffusion parameter of the standing stage; $D_2$ represents a diffusion parameter of the water pumping stage; $v_0$ represents a flow parameter of the water injection stage, $v_2$ represents a flow parameter of the water pumping stage, $v_0 > 0$, $v_2 < 0$, A represents an initial concentration of the groundwater pollutant, and $\partial\Omega$ represents derivative calculation along a boundary.

**6.** A groundwater pollutant control apparatus by a single-well pumping-injection process, comprising modules for carrying out the method steps of any of claims 1 to 5, wherein the apparatus comprises:

a parameter determination module (101) configured to determine first parameters, second parameters and third parameters of a to-be-controlled area; the first parameters characterizing parameters of a groundwater pollutant in a to-be-controlled area in a water injection stage, the second parameters characterizing parameters of the groundwater pollutant in a standing stage; and the third parameters characterizing hydraulic parameters of an aquifer in the to-be-controlled area and comprising water quality parameters reflecting inhomogeneity and anisotropy;

a parameter input module (102) configured to input the first parameters, the second parameters and the third parameters into a pumping-injection three-stage solute transport model to obtain fourth parameters output by the pumping-injection three-stage solute transport model; the pumping-injection three-stage solute transport model being trained on the basis of sample water quality parameters of an aquifer in a sample area and first sample pumping-injection parameters collected for a groundwater pollutant in the sample area in the water injection stage, the standing stage and a water pumping stage, and the water injection stage, the standing stage and the water pumping stage being performed in sequence; and the fourth parameters characterizing parameters of the groundwater pollutant in the water pumping stage and comprising a starting time, a duration and a water pumping speed of the water pumping stage; and

a plan determination module (103) configured to draw up a control plan for the to-be-controlled area on the basis of the fourth parameters;

wherein the pumping-injection three-stage solute transport model is established by the following modules:

a first determination module configured to determine the sample area on the basis of the third parameters;
a second determination module configured to determine the sample water quality parameters of the sample area; the sample water quality parameters being the water quality parameters reflecting inhomogeneity and anisotropy;
a third determination module configured to determine the first sample pumping-injection parameters; the first sample pumping-injection parameters comprising a starting time, a duration, a water injection speed and a water injection volume of the groundwater pollutant in the water injection stage, a duration of the standing stage as well as the starting time, the duration and the water pumping speed of the water pumping stage;

a first training module configured to use the sample water quality parameters and the first sample pumping-injection parameters as parameters used for establishment to establish the pumping-injection three-stage solute transport model; the pumping-injection three-stage solute transport model being a fractional derivative equation established under polar coordinates by stage-dividing on the basis of three stages comprising the water injection stage, the standing stage and the water pumping stage;

the pumping-injection three-stage solute transport model is:

$$\begin{cases} \frac{\partial C(r,t)}{\partial t} + \beta_0 \, {}^{TC}_0 D_t^{\alpha_0,\lambda_0} C(r,t) = -\frac{v_0}{r}\frac{\partial C(r,t)}{\partial r} + \frac{D_0}{r}\frac{\partial^2 C(r,t)}{\partial r^2}, t \in [0,T_1] \\ \frac{\partial C(r,t)}{\partial t} + \beta_1 \, {}^{TC}_{T_1} D_t^{\alpha_1,\lambda_1} C(r,t) = \frac{D_1}{r}\frac{\partial^2 C(r,t)}{\partial r^2}, t \in [T_1,T_2] \\ \frac{\partial C(r,t)}{\partial t} + \beta_2 \, {}^{TC}_{T_2} D_t^{\alpha_2,\lambda_2} C(r,t) = -\frac{v_2}{r}\frac{\partial C(r,t)}{\partial r} + \frac{D_2}{r} \, {}^{\alpha_1}_0 D_r^{\gamma} C(r,t), t \in [T_2,T_3] \end{cases}$$

wherein r represents a transport radius of the groundwater pollutant; t represents a transport time of the groundwater pollutant; C represents a concentration of the groundwater pollutant; $[0,T_1]$ represents the duration of the water injection stage; $[T_1,T_2]$ represents the duration of the standing stage; $[T_2,T_3]$ represents the duration of the water pumping stage; $T_3$ represents the sum of the durations of the water injection stage, the standing stage and the water pumping stage; $\beta_0$ represents a fractional-order capacity coefficient of the water injection stage; $\beta_1$: represents a fractional-order capacity coefficient of the standing stage; $\beta_2$ represents a fractional-order capacity coefficient of the water pumping stage; $\lambda_0$ represents a truncation coefficient of the water injection stage; $\lambda_1$ represents a truncation coefficient of the standing stage; $\lambda_2$ represents a truncation coefficient of the water pumping stage; $\alpha_0$ represents a fractional order of the water injection stage; $\alpha_1$ represents a fractional order of the standing stage; $\alpha_2$ represents a temporal fractional order of the water pumping stage; $\gamma$ represents a spatial fractional order of the water pumping stage, $0 < \alpha_0$, $\alpha_1, \alpha_2 \leq 1$, $1 < \gamma \leq 2$, $\alpha_0, \alpha_1, \alpha_2$ and $\gamma$ are all determined on the basis of the inhomogeneity of the sample water quality parameters and are in negative correlation with the inhomogeneity of the sample water quality parameters as well as the inhomogeneity of each of the sample water quality parameters; $D_0$ represents a diffusion parameter of the water injection stage; $D_1$: represents a diffusion parameter of the standing stage; $D_2$ represents a diffusion parameter of the water pumping stage; $v_0$ represents a flow parameter of the water injection stage, $v_2$ represents a flow parameter of the water pumping stage, $v_0 > 0$, and $v_2 < 0$.

7. An electronic device, **characterized by** comprising a memory, a processor, and a computer program stored in the memory and capable of running on the processor, wherein the processor, when executing the program, implements the steps of the method of any one of claims 1 to 5.

8. A non-transitory computer-readable storage medium, with a computer program stored therein, **characterized in that** the computer program, when executed by a processor, implements the steps of the method of any one of claims 1 to 5.

**Patentansprüche**

1. Computerimplementiertes Verfahren zur Kontrolle von Grundwasserschadstoffen durch einen Pumpeinspritzungsprozess mit einer einzigen Bohrung, wobei das Verfahren umfasst:

Bestimmen erster Parameter, zweiter Parameter und dritter Parameter eines zu kontrollierenden Bereichs, wobei die ersten Parameter Parameter eines Grundwasserschadstoffs in dem zu kontrollierenden Bereich in einer Wassereinspritzungsphase kennzeichnen, die zweiten Parameter Parameter des Grundwasserschadstoffs in einer Standphase kennzeichnen und die dritten Parameter hydraulische Parameter eines Aquifers in dem zu kontrollierenden Bereich kennzeichnen und Wasserqualitätsparameter umfassen, die eine Inhomogenität und eine Anisotropie widerspiegeln;
Eingeben der ersten Parameter, der zweiten Parameter und der dritten Parameter in ein dreiphasiges Pumpeinspritzungs-Soluttransportmodell, um vierte Parameter zu erhalten, die von dem dreiphasigen Pumpeinspritzungs-Soluttransportmodell ausgegeben werden, wobei das dreiphasige Pumpeinspritzungs-Soluttransportmodell auf Grundlage von Proben-Wasserqualitätsparametern eines Aquifers in einem Probenbereich und ersten Proben-Pumpeinspritzungsparametern trainiert ist, die für einen Grundwasserschadstoff in dem Probenbereich in der Wassereinspritzungsphase, der Standphase und einer Wasserförderphase erfasst werden, wobei die Wassereinspritzungsphase, die Standphase und die Wasserförderphase nacheinander durchgeführt werden, und wobei die vierten Parameter Parameter des Grundwasserschadstoffs in der Wasserförderphase kennzeichnen und einen Startzeitpunkt, eine Dauer und eine Wasserfördergeschwindigkeit der Wasserförderphase umfassen; und

Erstellen eines Kontrollplans für den zu kontrollierenden Bereich auf Grundlage der vierten Parameter;
wobei das dreiphasige Pumpeinspritzungs-Soluttransportmodell durch die folgenden Schritte erstellt wird:

Bestimmen des Probenbereichs auf Grundlage der dritten Parameter;
Bestimmen der Proben-Wasserqualitätsparameter des Probenbereichs, wobei die Proben-Wasserqualitätsparameter die Wasserqualitätsparameter sind, die Inhomogenität und Anisotropie widerspiegeln;
Bestimmen der ersten Proben-Pumpeinspritzungsparameter, wobei die ersten Proben-Pumpeinspritzungsparameter einen Startzeitpunkt, eine Dauer, eine Wassereinspritzungsgeschwindigkeit und ein Wassereinspritzungsvolumen des Grundwasserschadstoffs in der Wassereinspritzungsphase, eine Dauer der Standphase sowie den Startzeitpunkt, die Dauer und die Wasserfördergeschwindigkeit der Wasserförderphase umfassen;
Verwenden der Proben-Wasserqualitätsparameter und der ersten Proben-Pumpeinspritzungsparameter als zum Erstellen verwendete Parameter, um das dreiphasige Pumpeinspritzungs-Soluttransportmodell zu erstellen, wobei das dreiphasige Pumpeinspritzungs-Soluttransportmodell eine Gleichung mit fraktionalen Ableitungen ist, die in Polarkoordinaten durch phasenweise Unterteilung auf Grundlage von drei Phasen erstellt wird, wobei die drei Phasen die Wassereinspritzungsphase, die Standphase und die Wasserförderphase umfassen;
wobei das dreiphasige Pumpeinspritzungs-Soluttransportmodell lautet:

$$\begin{cases} \dfrac{\partial C(r,t)}{\partial t} + \beta_0 \, {}^{T_0^C}D_t^{\alpha_0,\lambda_0} C(r,t) = -\dfrac{v_0}{r}\dfrac{\partial C(r,t)}{\partial r} + \dfrac{D_0}{r}\dfrac{\partial^2 C(r,t)}{\partial r^2}, t \in [0,T_1] \\[2mm] \dfrac{\partial C(r,t)}{\partial t} + \beta_1 \, {}^{T_1^C}D_t^{\alpha_1,\lambda_1} C(r,t) = \dfrac{D_1}{r}\dfrac{\partial^2 C(r,t)}{\partial r^2}, t \in [T_1,T_2] \\[2mm] \dfrac{\partial C(r,t)}{\partial t} + \beta_2 \, {}^{T_2^C}D_t^{\alpha_2,\lambda_2} C(r,t) = -\dfrac{v_2}{r}\dfrac{\partial C(r,t)}{\partial r} + \dfrac{D_2}{r}\, {}^{R_0^L}D_r^{\gamma} C(r,t), t \in [T_2,T_3] \end{cases}$$

wobei $r$ einen Transportradius des Grundwasserschadstoffs darstellt, t eine Transportzeit des Grundwasserschadstoffs darstellt, C eine Konzentration des Grundwasserschadstoffs darstellt, $[0, T_1]$ die Dauer der Wassereinspritzungsphase darstellt, $[T_1, T_2]$ die Dauer der Standphase darstellt, $[T_2, T_3]$ die Dauer der Wasserförderphase darstellt, $T_3$ die Summe der Dauern der Wassereinspritzungsphase, der Standphase und der Wasserförderphase darstellt, $\beta_0$ einen Kapazitätskoeffizienten fraktionaler Ordnung der Wassereinspritzungsphase darstellt, $\beta_1$ einen Kapazitätskoeffizienten fraktionaler Ordnung der Standphase darstellt, $\beta_2$ einen Kapazitätskoeffizienten fraktionaler Ordnung der Wasserförderphase darstellt, $\lambda_0$ einen Trunkierungskoeffizienten der Wassereinspritzungsphase darstellt, $\lambda_1$ einen Trunkierungskoeffizienten der Standphase darstellt, $\lambda_2$ einen Trunkierungskoeffizienten der Wasserförderphase darstellt, $\alpha_0$ eine fraktionale Ordnung der Wassereinspritzungsphase darstellt, $\alpha_1$ eine fraktionale Ordnung der Standphase darstellt, $\alpha_2$ eine zeitliche fraktionale Ordnung der Wasserförderphase darstellt, $\gamma$ eine räumliche fraktionale Ordnung der Wasserförderphase darstellt, und wobei $0 < \alpha_0, \alpha_1, \alpha_2 \le 1$, $1 < \gamma \le 2$, und wobei $\alpha_0, \alpha_1, \alpha_2$ und $\gamma$ jeweils auf Grundlage der Inhomogenität der Proben-Wasserqualitätsparameter bestimmt werden und mit der Inhomogenität der Proben-Wasserqualitätsparameter sowie der Inhomogenität jedes der Proben-Wasserqualitätsparameter negativ korreliert sind, $D_0$ einen Diffusionsparameter der Wassereinspritzungsphase darstellt, $D_1$ einen Diffusionsparameter der Standphase darstellt, $D_2$ einen Diffusionsparameter der Wasserförderphase darstellt, $v_0$ einen Strömungsparameter der Wassereinspritzungsphase darstellt und $v_2$ einen Strömungsparameter der Wasserförderphase darstellt, und wobei $v_0 > 0$ und $v_2 < 0$.

2. Verfahren nach Anspruch 1, **dadurch gekennzeichnet, dass** der Schritt des Bestimmens der ersten Parameter, der zweiten Parameter und der dritten Parameter eines zu kontrollierenden Bereichs umfasst:

Bestimmen der dritten Parameter auf Grundlage von Explorationsdaten des zu kontrollierenden Bereichs, wobei die dritten Parameter Wasserqualitätsparameter umfassen, die eine Inhomogenität und eine Anisotropie widerspiegeln; und
Bestimmen der ersten Parameter und der zweiten Parameter, wobei die ersten Parameter den Startzeitpunkt, die Dauer, die Wassereinspritzungsgeschwindigkeit und das Wassereinspritzungsvolumen der Wassereinspritzungsphase umfassen und die zweiten Parameter die Dauer der Standphase sind.

3. Verfahren nach Anspruch 1, **dadurch gekennzeichnet, dass** das dreiphasige Pumpeinspritzungs-Soluttransportmodell auf Grundlage der Proben-Wasserqualitätsparameter des Aquifers in dem Probenbereich und zweiter Proben-Pumpeinspritzungsparameter trainiert ist, die für einen in den Probenbereich eingespritzten Wassertracer in der Wassereinspritzungsphase, der Standphase und der Wasserförderphase des Aquifers erfasst werden; wobei das dreiphasige Pumpeinspritzungs-Soluttransportmodell entsprechend ferner durch Umfassen der folgenden Schritte erstellt wird:

Einspritzen des Wassertracers in den Probenbereich und Bestimmen der zweiten Proben-Pumpeinspritzungsparameter, wobei die zweiten Proben-Pumpeinspritzungsparameter einen Startzeitpunkt, eine Dauer, eine Wassereinspritzungsgeschwindigkeit und ein Wassereinspritzungsvolumen des Tracers in der Wassereinspritzungsphase, eine Dauer der Standphase sowie einen Startzeitpunkt, eine Dauer und eine Wasserfördergeschwindigkeit der Wasserförderphase umfassen; und
Verwenden der Proben-Wasserqualitätsparameter und der zweiten Proben-Pumpeinspritzungsparameter als zum Erstellen verwendete Parameter, um das dreiphasige Pumpeinspritzungs-Soluttransportmodell zu erstellen, wobei das dreiphasige Pumpeinspritzungs-Soluttransportmodell eine Gleichung mit fraktionalen Ableitungen ist, die in Polarkoordinaten durch phasenweise Unterteilung auf Grundlage von drei Phasen erstellt wird, wobei die drei Phasen die Wassereinspritzungsphase, die Standphase und die Wasserförderphase umfassen.

4. Verfahren nach Anspruch 3, **dadurch gekennzeichnet, dass** das dreiphasige Pumpeinspritzungs-Soluttransportmodell ferner auf Grundlage von Betriebsbedingungsparametern des Probenbereichs trainiert ist, wobei die Betriebsbedingungsparameter eine Anfangskonzentration und Randbedingungen des Grundwasserschadstoffs umfassen; wobei das dreiphasige Pumpeinspritzungs-Soluttransportmodell entsprechend durch die folgenden Schritte erstellt wird:

Bestimmen des Probenbereichs auf Grundlage der dritten Parameter;
Bestimmen der Proben-Wasserqualitätsparameter und der Betriebsbedingungsparameter des Probenbereichs;
Bestimmen der ersten Proben-Pumpeinspritzungsparameter, wobei die ersten Proben-Pumpeinspritzungsparameter den Startzeitpunkt, die Dauer, die Wassereinspritzungsgeschwindigkeit und das Wassereinspritzungsvolumen der Wassereinspritzungsphase, die Dauer der Standphase sowie den Startzeitpunkt, die Dauer und die Wasserfördergeschwindigkeit der Wasserförderphase umfassen; und Verwenden der Proben-Wasserqualitätsparameter, der ersten Proben-Pumpeinspritzungsparameter und der Betriebsbedingungsparameter als zum Erstellen verwendete Parameter, um das dreiphasige Pumpeinspritzungs-Soluttransportmodell zu erstellen, wobei das dreiphasige Pumpeinspritzungs-Soluttransportmodell eine Gleichung mit fraktionalen Ableitungen ist, die in Polarkoordinaten durch phasenweise Unterteilung auf Grundlage von drei Phasen erstellt wird, wobei die drei Phasen die Wassereinspritzungsphase, die Standphase und die Wasserförderphase umfassen.

5. Verfahren nach Anspruch 4, **dadurch gekennzeichnet, dass** das dreiphasige Pumpeinspritzungs-Soluttransportmodell lautet:

$$\begin{cases} \frac{\partial C(r,t)}{\partial t} + \beta_0 \, {}^{TC}_0 D_t^{\alpha_0,\lambda_0} C(r,t) = -\frac{v_0}{r}\frac{\partial C(r,t)}{\partial r} + \frac{D_0}{r}\frac{\partial^2 C(r,t)}{\partial r^2}, t \in [0, T_1] \\ \frac{\partial C(r,t)}{\partial t} + \beta_1 \, {}^{TC}_{T_1} D_t^{\alpha_1,\lambda_1} C(r,t) = \frac{D_1}{r}\frac{\partial^2 C(r,t)}{\partial r^2}, t \in [T_1, T_2] \\ \frac{\partial C(r,t)}{\partial t} + \beta_2 \, {}^{TC}_{T_2} D_t^{\alpha_2,\lambda_2} C(r,t) = -\frac{v_2}{r}\frac{\partial C(r,t)}{\partial r} + \frac{D_2}{r} \, {}^{RL}_0 D_r^{\gamma} C(x,t), t \in [T_2, T_3] \\ \frac{\partial C(r,t)}{\partial r}\Big|_{\partial\Omega} = 0 \\ C(r,0) = \begin{cases} A, r = 0 \\ 0, \text{sonst} \end{cases}, C(r, T_1^+) = C(r, T_1^-), C(r, T_2^+) = C(r, T_2^-) \end{cases}$$

wobei r einen Transportradius des Grundwasserschadstoffs darstellt, t eine Transportzeit des Grundwasserschadstoffs darstellt, C eine Konzentration des Grundwasserschadstoffs darstellt, $[0, T_1]$ die Dauer der Wassereinspritzungsphase darstellt, $[T_1, T_2]$ die Dauer der Standphase darstellt, $[T_2, T_3]$ die Dauer der Wasserförderphase darstellt, $T_3$ die Summe der Dauern der Wassereinspritzungsphase, der Standphase und der Wasserförderphase darstellt, $\beta_0$ einen Kapazitätskoeffizienten fraktionaler Ordnung der Wassereinspritzungsphase darstellt, $\beta_1$ einen Kapazitätskoeffizienten fraktionaler Ordnung der Standphase darstellt, $\beta_2$ einen Kapazitätskoeffizienten fraktionaler Ordnung der Wasserförderphase darstellt, $\lambda_0$ einen Trunkierungskoeffizienten der Wassereinspritzungsphase darstellt, $\lambda_1$ einen Trunkierungskoeffizienten der Standphase darstellt, $\lambda_2$ einen Trunkierungskoeffizienten der Wasserförderphase darstellt, $\alpha_0$ eine fraktionale Ordnung der Wassereinspritzungsphase darstellt, $\alpha_1$ eine fraktionale Ordnung der Standphase darstellt, $\alpha_2$ eine zeitliche fraktionale Ordnung der Wasserförderphase darstellt, $\gamma$ eine räumliche fraktionale Ordnung der Wasserförderphase darstellt, und wobei $0 < \alpha_0$, $\alpha_1$, $\alpha_2 \leq 1$, $1 < \gamma \leq 2$, und wobei $\alpha_0$, $\alpha_1$, $\alpha_2$ und $\gamma$ jeweils auf Grundlage der Inhomogenität der Proben-Wasserqualitätsparameter bestimmt werden und mit der Inhomogenität der Proben-Wasserqualitätsparameter sowie mit der Inhomogenität jedes der Proben-Wasserqualitätsparameter negativ korreliert sind, $D_0$ einen Diffusionsparameter der Wassereinspritzungsphase darstellt, $D_1$ einen Diffusionsparameter der Standphase darstellt, $D_2$ einen Diffusionsparameter der Wasserförderphase darstellt, $v_0$ einen Strömungsparameter der Wassereinspritzungsphase darstellt, $v_2$ einen Strömungsparameter der Wasserförderphase darstellt, und wobei $v_0 > 0$ und $v_2 < 0$, und wobei $A$ eine Anfangskonzentration des Grundwasserschadstoffs darstellt und $\partial\Omega$ eine Ableitungsberechnung entlang einer Grenze darstellt.

6. Vorrichtung zur Kontrolle von Grundwasserschadstoffen durch einen Pumpeinspritzungsprozess mit einer einzigen Bohrung, umfassend Module zum Ausführen der Verfahrensschritte nach einem der Ansprüche 1 bis 5, wobei die Vorrichtung umfasst:

ein Parameterbestimmungsmodul (101), das dazu konfiguriert ist, erste Parameter, zweite Parameter und dritte Parameter eines zu kontrollierenden Bereichs zu bestimmen, wobei die ersten Parameter Parameter eines Grundwasserschadstoffs in einem zu kontrollierenden Bereich in einer Wassereinspritzungsphase kennzeichnen, die zweiten Parameter Parameter des Grundwasserschadstoffs in einer Standphase kennzeichnen und die dritten Parameter hydraulische Parameter eines Aquifers in dem zu kontrollierenden Bereich kennzeichnen und Wasserqualitätsparameter umfassen, die Inhomogenität und Anisotropie widerspiegeln;
ein Parametereingabemodul (102), das dazu konfiguriert ist, die ersten Parameter, die zweiten Parameter und die dritten Parameter in ein dreiphasiges Pumpeinspritzungs-Soluttransportmodell einzugeben, um vierte Parameter zu erhalten, die von dem dreiphasigen Pumpeinspritzungs-Soluttransportmodell ausgegeben werden, wobei das dreiphasige Pumpeinspritzungs-Soluttransportmodell auf Grundlage von Proben-Wasserqualitätsparametern eines Aquifers in einem Probenbereich und ersten Proben-Pumpeinspritzungsparametern trainiert ist, die für einen Grundwasserschadstoff in dem Probenbereich in der Wassereinspritzungsphase, der Standphase und einer Wasserförderphase erfasst werden, und wobei die Wassereinspritzungsphase, die Standphase und die Wasserförderphase nacheinander durchgeführt werden, und wobei die vierten Parameter Parameter des Grundwasserschadstoffs in der Wasserförderphase kennzeichnen und einen Startzeitpunkt, eine Dauer und eine Wasserfördergeschwindigkeit der Wasserförderphase umfassen; und
ein Planbestimmungsmodul (103), das dazu konfiguriert ist, auf Grundlage der vierten Parameter einen Kontrollplan für den zu kontrollierenden Bereich zu erstellen;
wobei das dreiphasige Pumpeinspritzungs-Soluttransportmodell durch die folgenden Module erstellt wird:

ein erstes Bestimmungsmodul, das dazu konfiguriert ist, den Probenbereich auf Grundlage der dritten Parameter zu bestimmen;
ein zweites Bestimmungsmodul, das dazu konfiguriert ist, die Proben-Wasserqualitätsparameter des Probenbereichs zu bestimmen, wobei die Proben-Wasserqualitätsparameter die Wasserqualitätsparameter sind, die Inhomogenität und Anisotropie widerspiegeln;
ein drittes Bestimmungsmodul, das dazu konfiguriert ist, die ersten Proben-Pumpeinspritzungsparameter zu bestimmen, wobei die ersten Proben-Pumpeinspritzungsparameter einen Startzeitpunkt, eine Dauer, eine Wassereinspritzungsgeschwindigkeit und ein Wassereinspritzungsvolumen des Grundwasserschadstoffs in der Wassereinspritzungsphase, eine Dauer der Standphase sowie den Startzeitpunkt, die Dauer und die Wasserfördergeschwindigkeit der Wasserförderphase umfassen;
ein erstes Trainingsmodul, das dazu konfiguriert ist, die Proben-Wasserqualitätsparameter und die ersten Proben-Pumpeinspritzungsparameter als zum Erstellen verwendete Parameter zu verwenden, um das dreiphasige Pumpeinspritzungs-Soluttransportmodell zu erstellen, wobei das dreiphasige Pumpeinspritzungs-Soluttransportmodell eine Gleichung mit fraktionalen Ableitungen ist, die in Polarkoordinaten durch phasenweise Unterteilung auf Grundlage von drei Phasen erstellt wird, wobei die drei Phasen die Wassereinspritzungsphase, die Standphase und die Wasserförderphase umfassen;

wobei das dreiphasige Pumpeinspritzungs-Soluttransportmodell lautet:

$$\begin{cases} \dfrac{\partial C(r,t)}{\partial t} + \beta_0\, {}^{T\varsigma}_0 D_t^{\alpha_0,\lambda_0} C(r,t) = -\dfrac{v_0}{r}\dfrac{\partial C(r,t)}{\partial r} + \dfrac{D_0}{r}\dfrac{\partial^2 C(r,t)}{\partial r^2},\ t \in [0,T_1] \\[2mm] \dfrac{\partial C(r,t)}{\partial t} + \beta_1\, {}^{T\varsigma}_{T_1} D_t^{\alpha_1,\lambda_1} C(r,t) = \dfrac{D_1}{r}\dfrac{\partial^2 C(r,t)}{\partial r^2},\ t \in [T_1,T_2] \\[2mm] \dfrac{\partial C(r,t)}{\partial t} + \beta_2\, {}^{T\varsigma}_{T_2} D_t^{\alpha_2,\lambda_2} C(r,t) = -\dfrac{v_2}{r}\dfrac{\partial C(r,t)}{\partial r} + \dfrac{D_2}{r}\, {}^{RL}_0 D_r^{\gamma} C(r,t),\ t \in [T_2,T_3] \end{cases}$$

wobei r einen Transportradius des Grundwasserschadstoffs darstellt, *t* eine Transportzeit des Grundwasserschadstoffs darstellt, C eine Konzentration des Grundwasserschadstoffs darstellt, $[0, T_1]$ die Dauer der Wassereinspritzungsphase darstellt, $[T_1, T_2]$ die Dauer der Standphase darstellt, $[T_2, T_3]$ die Dauer der Wasserförderphase darstellt, $T_3$ die Summe der Dauern der Wassereinspritzungsphase, der Standphase und der Wasserförderphase darstellt, $\beta_0$ einen Kapazitätskoeffizienten fraktionaler Ordnung der Wassereinspritzungsphase darstellt, $\beta_1$ einen Kapazitätskoeffizienten fraktionaler Ordnung der Standphase darstellt, $\beta_2$ einen Kapazitätskoeffizienten fraktionaler Ordnung der Wasserförderphase darstellt, $\lambda_0$ einen Trunkierungskoeffizienten der Wassereinspritzungsphase darstellt, $\lambda_1$ einen Trunkierungskoeffizienten der Standphase darstellt, $\lambda_2$ einen Trunkierungskoeffizienten der Wasserförderphase darstellt, $\alpha_0$ eine fraktionale Ordnung der Wassereinspritzungsphase darstellt, $\alpha_1$ eine fraktionale Ordnung der Standphase darstellt, $\alpha_2$ eine zeitliche fraktionale Ordnung der Wasserförderphase darstellt, $\gamma$ eine räumliche fraktionale Ordnung der Wasserförderphase darstellt, und wobei $0 < \alpha_0, \alpha_1, \alpha_2 \leq 1$, $1 < \gamma \leq 2$, und wobei $\alpha_0, \alpha_1, \alpha_2$ und $\gamma$ jeweils auf Grundlage der Inhomogenität der Proben-Wasserqualitätsparameter bestimmt werden und mit der Inhomogenität der Proben-Wasserqualitätsparameter sowie mit der Inhomogenität jedes der Proben-Wasserqualitätsparameter negativ korreliert sind, $D_0$ einen Diffusionsparameter der Wassereinspritzungsphase darstellt, $D_1$ einen Diffusionsparameter der Standphase darstellt, $D_2$ einen Diffusionsparameter der Wasserförderphase darstellt, $v_0$ einen Strömungsparameter der Wassereinspritzungsphase darstellt und $v_2$ einen Strömungsparameter der Wasserförderphase darstellt, und wobei $v_0 > 0$ und $v_2 < 0$.

7. Elektronische Vorrichtung, **dadurch gekennzeichnet, dass** sie einen Speicher, einen Prozessor und ein in dem Speicher gespeichertes und auf dem Prozessor lauffähiges Computerprogramm umfasst, wobei der Prozessor beim Ausführen des Programms die Schritte des Verfahrens nach einem der Ansprüche 1 bis 5 implementiert.

8. Nichtflüchtiges computerlesbares Speichermedium, auf dem ein Computerprogramm gespeichert ist, **dadurch gekennzeichnet, dass** das Computerprogramm, wenn es von einem Prozessor ausgeführt wird, die Schritte des Verfahrens nach einem der Ansprüche 1 bis 5 implementiert.

**Revendications**

1. Procédé de contrôle des polluants phréatiques implémentée par ordinateur par un procédé d'injection par pompage à puits unique, le procédé comprenant :

déterminer les premiers, les deuxièmes et les troisièmes paramètres d'une zone à contrôler ; les premiers paramètres caractérisant les paramètres d'un polluant phréatique dans une zone à contrôler dans une étape d'injection d'eau, les deuxièmes paramètres caractérisant les paramètres du polluant phréatique dans une étape fixe ; et le troisième paramètre caractérisant les paramètres hydrauliques d'un aquifère dans la zone à contrôler et comprenant des paramètres de qualité de l'eau reflétant l'inhomogénéité et l'anisotropie ;
saisir les premiers, les deuxièmes et les troisièmes paramètres dans un modèle de transport de soluté à trois étapes d'injection par pompage afin d'obtenir la sortie des quatrièmes paramètres par le modèle de transport de soluté à trois étapes d'injection par pompage ; le modèle de transport de soluté à trois étapes d'injection par pompage entraîné sur la base des paramètres de qualité de l'eau d'un échantillon d'aquifère dans une zone d'échantillonnage et des premiers paramètres d'injection par pompage collectés pour un polluant d'eau souterraine dans la zone d'échantillonnage à l'étape d'injection d'eau, à l'étape stationnaire et à l'étape de pompage d'eau, et à l'étape d'injection d'eau, à l'étape stationnaire et à l'étape de pompage d'eau réalisés en séquence ; et les quatrièmes paramètres caractérisant les paramètres du polluant phréatique dans l'étape de pompage et comprenant un temps de début, une durée et une vitesse de pompage d'eau de l'étape de pompage d'eau ; et

l'élaboration d'un plan de contrôle pour la zone à contrôler sur la base des quatrièmes paramètres ;

dans lequel le modèle de transport du soluté à trois étapes d'injection par pompage est établi par les étapes suivantes :

déterminer la zone d'échantillonnage sur la base des troisièmes paramètres ;

déterminer les paramètres de qualité de l'eau de l'échantillon dans la zone d'échantillonnage ; les paramètres de qualité de l'eau de l'échantillon étant les paramètres de qualité de l'eau reflétant l'inhomogénéité et l'anisotropie ;

déterminer les premiers paramètres d'injection par pompage de l'échantillon ; les premiers paramètres d'injection par pompage comprenant un temps de début, une durée, une vitesse d'injection d'eau et un volume d'injection d'eau du polluant phréatique dans l'étape d'injection, une durée de l'étape stationnaire ainsi que le temps de début, la durée et la vitesse de ping d'eau de l'étape de pompage d'eau ;

utiliser les paramètres de qualité de l'eau de l'échantillon et les premiers paramètres d'injection par pompage comme paramètres d'établissement pour établir le modèle de transport de soluté à trois étapes d'injection par pompage ; le modèle de transport de soluté à trois étapes d'injection par pompage est une équation de dérivée fractionnaire établie selon des coordonnées polaires par division d'étapes sur la base de trois étapes comprenant l'étape d'injection d'eau, l'étape stationnaire et l'étape de pompage d'eau ;

dans lequel le modèle de transport de soluté à trois étapes d'injection par pompage est :

$$\begin{cases} \dfrac{\partial C(r,t)}{\partial t} + \beta_0\,{}^{TC}_0 D_t^{\alpha_0,\lambda_0} C(r,t) = -\dfrac{v_0}{r}\dfrac{\partial C(r,t)}{\partial r} + \dfrac{D_0}{r}\dfrac{\partial^2 C(r,t)}{\partial r^2},\, t \in [0,T_1] \\[2mm] \dfrac{\partial C(r,t)}{\partial t} + \beta_1\,{}^{TC}_{T_1} D_t^{\alpha_1,\lambda_1} C(r,t) = \dfrac{D_1}{r}\dfrac{\partial^2 C(r,t)}{\partial r^2},\, t \in [T_1,T_2] \\[2mm] \dfrac{\partial C(r,t)}{\partial t} + \beta_2\,{}^{TC}_{T_2} D_t^{\alpha_2,\lambda_2} C(r,t) = -\dfrac{v_2}{r}\dfrac{\partial C(r,t)}{\partial r} + \dfrac{D_2}{r}\,{}^{RL}_0 D_r^{\gamma} C(r,t),\, t \in [T_2,T_3] \end{cases}$$

où r représente un rayon de transport du polluant phréatique ; t représente le temps de transport du polluant phréatique ; C représente une concentration de polluant phréatique ; $[0,T_1]$ représente la durée de l'étape d'injection d'eau ; $[T_1,T_2]$ représente la durée de l'étape stationnaire ; $[T_2,T_3]$ représente la durée de l'étape de pompage de l'eau ; $T_3$ représente la somme des durées de l'étape d'injection d'eau, de l'étape stationnaire et de l'étape de pompage ; $\beta_0$ représente un coefficient de capacité d'ordre fractionnaire de l'étape d'injection d'eau ; $\beta_1$ représente un coefficient de capacité d'ordre fractionnaire de l'étape stationnaire ; $\beta_2$ représente un coefficient de capacité d'ordre fractionnaire de l'étape de pompage de l'eau ; $\lambda_0$ représente un coefficient de troncature de l'étape d'injection d'eau ; $\lambda_1$ représente un coefficient de troncature de l'étape stationnaire ; $\lambda_2$ représente un coefficient de troncature de l'étape de pompage de l'eau ; $\alpha_0$ représente un ordre fractionnaire de l'étape d'injection d'eau ; $\alpha_1$ représente un ordre fractionnaire de l'étape stationnaire ; $\alpha_2$ représente un ordre fractionnaire temporel de l'étape de pompage de l'eau ; $\gamma$ représente un ordre fractionnaire spatial de l'étape de pompage de l'eau, $0 < \alpha_0,\ \alpha_1,\ \alpha_2 \leq 1$, $1 < \gamma \leq 2$, $\alpha_0,\ \alpha_1,\ \alpha_2$ et $Y$ sont tous déterminés sur la base de l'inhomogénéité des paramètres de qualité de l'eau de l'échantillon et sont en corrélation négative avec l'inhomogénéité des paramètres de qualité de l'eau de l'échantillon ainsi que l'inhomogénéité de chacun des paramètres de qualité de l'eau de l'échantillon ; $D_0$ représente un paramètre de diffusion de l'étape d'injection d'eau ; $D_1$ représente un paramètre de diffusion de l'étape stationnaire ; $D_2$ représente un paramètre de diffusion de l'étape de pompage de l'eau ; $v_0$ représente un paramètre de débit de l'étape d'injection d'eau, $v_2$ représente un paramètre de débit de l'étape de pompage d'eau. $v_0 > 0$, et $v_2 < 0$.

2. Procédé selon la revendication 1, **caractérisé en ce que** la détermination des premiers, des deuxièmes et des troisièmes paramètres d'une zone à contrôler, comprend :

déterminer les troisièmes paramètres sur la base des données d'exploration de la zone à contrôler ; les troisièmes paramètres comprenant la qualité de l'eau reflétant l'inhomogénéité et l'anisotropie ; et

déterminer les premiers paramètres et les deuxièmes paramètres ; les premiers paramètres comprenant le temps de début, la durée, la vitesse d'injection d'eau et le volume d'injection d'eau de l'étape d'injection d'eau ; et les deuxièmes paramètres étant la durée de la phase stationnaire.

3. Procédé selon la revendication 1, **caractérisé en ce que** le modèle de transport de soluté à trois étapes d'injection par pompage est entraîné sur la base des paramètres de qualité de l'eau de l'échantillon de l'aquifère dans la zone d'échantillonnage et des deuxièmes paramètres d'injection par pompage collectés pour un traceur d'eau injecté dans la zone d'échantillonnage à l'étape d'injection d'eau, à l'étape stationnaire et à l'étape de pompage de l'aquifère ; en conséquence, le modèle de transport de solutés à trois étapes d'injection par pompage est établi en comprenant en outre les étapes suivantes :

injecter le traceur d'eau dans la zone d'échantillon, et déterminer les deuxièmes paramètres d'injection par pompage de l'échantillon ; les paramètres d'injection par pompage du deuxième échantillon comprennent un temps de début, une durée, une vitesse d'injection d'eau et un volume d'injection d'eau du traceur dans l'étape d'injection d'eau, une durée de l'étape stationnaire ainsi qu'un temps de début, une durée et une vitesse de pompage de l'étape de pompage ; et
utiliser les paramètres de qualité de l'eau de l'échantillon et les paramètres d'injection par pompage du deuxième échantillon comme paramètres d'établissement afin d'établir le modèle de transport de soluté à trois étapes d'injection par pompage ; le modèle de transport de soluté à trois étapes d'injection par pompage est une équation de dérivée fractionnaire établie sous coordonnées polaires par division d'étape sur la base de trois étapes comprenant l'étape d'injection d'eau, l'étape stationnaire et l'étape de pompage d'eau.

4.  Procédé selon la revendication 3, **caractérisé en ce que** le modèle de transport du soluté à trois étapes d'injection par pompage est entraîné en profondeur sur la base des paramètres de conditions de fonctionnement de la zone d'échantillon ; les paramètres de conditions de fonctionnement comprennent une concentration initiale et des conditions limites du polluant phréatique ;
en conséquence, le modèle de transport de soluté à trois étapes d'injection par pompage est établi par les étapes suivantes :

déterminer la zone d'échantillonnage sur la base des troisièmes paramètres ;
déterminer les paramètres de qualité de l'eau de l'échantillon et les paramètres de conditions de fonctionnement de la zone d'échantillonnage ;
déterminer les premiers paramètres d'injection par pompage de l'échantillon ; les premiers paramètres d'injection de pompage comprenant le temps de début, la durée, la vitesse d'injection d'eau et le volume d'injection d'eau de l'étape d'injection, la durée de l'étape stationnaire ainsi que le temps de début, la durée et la vitesse de pompage de l'eau ; et
utiliser les paramètres de qualité de l'eau de l'échantillon, les premiers paramètres d'injection par pompage de l'échantillon et les paramètres de conditions de fonctionnement comme paramètres d'établissement afin d'établir le modèle de transport de soluté à trois étapes d'injection par pompage ; le modèle de transport de soluté à trois étapes d'injection par pompage est une équation de dérivée fractionnaire établie sous coordonnées polaires par division d'étape sur la base de trois étapes comprenant l'étape d'injection d'eau, l'étape stationnaire et l'étape de pompage d'eau.

5.  Procédé selon la revendication 4, **caractérisé en ce que** le modèle de transport de solutés à trois étapes d'injection par pompage est :

$$\begin{cases} \frac{\partial C(r,t)}{\partial t} + \beta_0 \, {}^{TC}_0 D_t^{\alpha_0,\lambda_0} C(r,t) = -\frac{v_0}{r}\frac{\partial C(r,t)}{\partial r} + \frac{D_0}{r}\frac{\partial^T C(r,t)}{\partial r^2}, t \in [0,T_1] \\ \frac{\partial C(r,t)}{\partial t} + \beta_1 \, {}^{TC}_{T_1} D_r^{\alpha_1,\lambda_1} C(r,t) = \frac{D_1}{r}\frac{\partial^2 C(r,t)}{\partial r^2}, t \in [T_1,T_2] \\ \frac{\partial C(r,t)}{\partial t} + \beta_2 \, {}^{TC}_{T_2} D_t^{\alpha_2,\lambda_2} C(r,t) = -\frac{v_2}{r}\frac{\partial C(r,t)}{\partial r} + \frac{D_2}{r} \, {}^{RL}_0 D_r^{\gamma} C(x,t), t \in [T_2,T_3] \\ \frac{\partial C(r,t)}{\partial r}\Big|_{\partial\Omega} = 0 \\ C(r,0) = \begin{cases} A, r = 0 \\ 0, else \end{cases}, C(r,T_1^{+}) = C(r,T_1^{-}), C(r,T_2^{+}) = C(r,T_2^{-}) \end{cases}$$

où r représente un rayon de transport du polluant phréatique ; t représente le temps de transport du polluant phréatique ; C représente une concentration de polluant phréatique ; $[0,T_1]$ représente la durée de l'étape d'injection d'eau ; $[T_1,T_2]$ représente la durée de l'étape stationnaire ; $[T_2,T_3]$ représente la durée de l'étape de pompage de l'eau ; $T_3$ représente la somme des durées de l'étape d'injection d'eau, de l'étape stationnaire et de l'étape de pompage ; $\beta_0$ représente un coefficient de capacité d'ordre fractionnaire de l'étape d'injection d'eau ; $\beta_1$ : représente un coefficient de capacité d'ordre fractionnaire de l'étape stationnaire ; $\beta_2$ représente un coefficient de capacité d'ordre fractionnaire de l'étape de pompage de l'eau ; $\lambda_0$ représente un coefficient de troncature de l'étape d'injection d'eau ; $\lambda_1$ représente un coefficient de troncature de l'étape stationnaire ; $\lambda_2$ représente un coefficient de troncature de l'étape de pompage de l'eau ; $\alpha_0$ représente un ordre fractionnaire de l'étape d'injection d'eau ; $\alpha_1$ représente un ordre fractionnaire de

l'étape stationnaire ; $\alpha_2$ représente un ordre fractionnaire temporel de l'étape de pompage de l'eau ; $\gamma$ représente un ordre fractionnaire spatial de l'étape de pompage de l'eau, 0 < $\alpha_0$, $\alpha_1$, $\alpha_2$ ≤ 1, 1 < $\gamma$ ≤ 2, $\alpha_0$, $\alpha_1$ et $\alpha_2\gamma$ sont tous arrêtés sur la base de l'inhomogénéité des paramètres de qualité de l'eau de l'échantillon et sont en corrélation négative avec l'inhomogénéité des paramètres de qualité de l'eau de l'échantillon ainsi que l'inhomogénéité de chacun des paramètres de qualité de l'eau de l'échantillon ; $D_0$ représente un paramètre de diffusion de l'étape d'injection d'eau ; $D_1$ représente un paramètre de diffusion de l'étape stationnaire ; $D_2$ représente un paramètre de diffusion de l'étape de pompage de l'eau ; $v_0$ représente un paramètre de débit de l'étape d'injection d'eau, $v_2$ représente un paramètre de débit de l'étape de pompage d'eau. $v_0$>0, $v_1$<0, A représente une concentration initiale du polluant phréatique, et $\partial\Omega$ représente le calcul des dérivées le long d'une limite.

6. Appareil de contrôle des polluants phréatiques par un procédé d'injection par pompage à puits unique, comprenant des modules pour réaliser les étapes de procédé de l'une des revendications 1 à 5, dans lequel l'appareil comprend :

un module de détermination des paramètres (101) configuré pour déterminer les premiers, les deuxièmes et les troisièmes paramètres d'une zone à contrôler ; les premiers paramètres caractérisant les paramètres d'un polluant phréatique dans une zone à contrôler à l'étape d'injection d'eau, les deuxièmes paramètres caractérisant les paramètres du polluant phréatique à l'étape fixe ; et le troisième paramètre caractérisant les paramètres hydrauliques d'un aquifère dans la zone à contrôler et comprenant des paramètres de qualité de l'eau reflétant l'inhomogénéité et l'anisotropie ;

un module d'entrée de paramètres (102) configuré pour entrer les premiers, les deuxièmes et les troisièmes paramètres dans un modèle de transport de soluté à trois étapes d'injection par pompage afin d'obtenir la sortie des quatrièmes paramètres par le modèle de transport de soluté à trois étapes d'injection par pompage ; le modèle de transport de solutés à trois étapes d'injection par pompage entraîné sur la base des paramètres de qualité de l'eau d'un échantillon d'aquifère dans une zone d'échantillonnage et des premiers paramètres d'injection par pompage collectés pour un polluant phréatique dans la zone d'échantillonnage à l'étape d'injection d'eau, à l'étape stationnaire et à l'étape de pompage d'eau, et à l'étape d'injection d'eau, à l'étape stationnaire et à l'étape de pompage d'eau réalisés en séquence ; et le quatrième paramètre caractérisant les paramètres du polluant phréatique dans l'étape de pompage et comprenant un temps de début, une durée et une vitesse de pompage de l'étape ; et

un module de détermination de plan (103) configuré pour élaborer un plan de contrôle pour la zone à contrôler sur la base des quatrièmes paramètres ;

dans lequel le modèle de transport de soluté à trois étapes d'injection par pompage est établi par les modules suivants :

un premier module de détermination configuré pour déterminer la surface d'échantillonnage sur la base des troisièmes paramètres ;

un deuxième module de détermination configuré pour déterminer les paramètres de qualité de l'eau de l'échantillon dans la zone d'échantillonnage ; les paramètres de qualité de l'eau de l'échantillon étant les paramètres de qualité de l'eau reflétant l'inhomogénéité et l'anisotropie ;

un troisième module de détermination configuré pour déterminer les premiers paramètres d'injection par pompage de l'échantillon ; les premiers paramètres d'injection de pompage comprennent un temps de début, une durée, une vitesse d'injection d'eau et un volume d'injection d'eau du polluant souterrain dans l'étape d'injection, une durée de l'étape stationnaire ainsi que le temps de début, la durée et la vitesse de pompage de l'étape de pompage ;

un premier module d'entraînement configuré pour utiliser les paramètres de qualité de l'eau de l'échantillon et les premiers paramètres d'injection par pompage comme paramètres d'établissement afin d'établir le modèle de transport de solutés à trois étapes d'injection par pompage ; le modèle de transport de soluté à trois étapes d'injection par pompage est une équation de dérivée fractionnaire établie selon des coordonnées polaires par division d'étapes sur la base de trois étapes comprenant l'étape d'injection d'eau, l'étape stationnaire et l'étape de pompage d'eau ;

le modèle de transport de soluté à trois étapes d'injection par pompage est :

$$\begin{cases} \dfrac{\partial C(r,t)}{\partial t} + \beta_0 \,^{TC}_{0}D_t^{\alpha_0,\lambda_0} C(r,t) = -\dfrac{v_0}{r}\dfrac{\partial C(r,t)}{\partial r} + \dfrac{D_0}{r}\dfrac{\partial^2 C(r,t)}{\partial r^2}, t \in [0,T_1] \\[2mm] \dfrac{\partial C(r,t)}{\partial t} + \beta_1 \,^{TC}_{T_1}D_t^{\alpha_1,\lambda_1} C(r,t) = \dfrac{D_1}{r}\dfrac{\partial^2 C(r,t)}{\partial r^2}, t \in [T_1,T_2] \\[2mm] \dfrac{\partial C(r,t)}{\partial t} + \beta_2 \,^{TC}_{T_2}D_t^{\alpha_2,\lambda_2} C(r,t) = -\dfrac{v_2}{r}\dfrac{\partial C(r,t)}{\partial r} + \dfrac{D_2}{r}\,^{RL}_{0}D_r^{\gamma} C(r,t), t \in [T_2,T_3] \end{cases}$$

où r représente un rayon de transport du polluant phréatique ; t représente le temps de transport du polluant phréatique ; C représente une concentration de polluant phréatique ; $[0,T_1]$ représente la durée de l'étape d'injection d'eau ; $[T_1,T_2]$ représente la durée de l'étape stationnaire ; $[T_2,T_3]$ représente la durée de l'étape de pompage de l'eau ; $T_3$ représente la somme des durées de l'étape d'injection d'eau, représente la somme des durées de l'étape d'injection d'eau ; $\beta_0$ représente un coefficient de capacité d'ordre fractionnaire de l'étape d'injection d'eau ; $\beta_1$ représente un coefficient de capacité d'ordre fractionnaire de l'étape stationnaire ; $\beta_2$ représente un coefficient de capacité d'ordre fractionnaire de l'étape de pompage de l'eau ; $\lambda_0$ représente un coefficient de troncature de l'étape d'injection d'eau ; $\lambda_1$ représente un coefficient de troncature de l'étape stationnaire ; $\lambda_2$ représente un coefficient de troncature de l'étape de pompage de l'eau ; $\alpha_0$ représente un ordre fractionnaire de l'étape d'injection d'eau ; $\alpha_1$ représente un ordre fractionnaire de l'étape stationnaire ; $\alpha_2$ représente un ordre fractionnaire temporel de l'étape de pompage de l'eau ; $\gamma$ représente un ordre fractionnaire spatial de l'étape de pompage de l'eau, $0 < \alpha_0, \alpha_1, \alpha_2 \leq 1$, $1 < \gamma \leq 2$, $\alpha_0, \alpha_1, \alpha_2$ et $\gamma$ sont tous déterminés sur la base de l'inhomogénéité des paramètres de qualité de l'eau de l'échantillon et sont en corrélation négative avec l'inhomogénéité des paramètres de qualité de l'eau de l'échantillon ainsi que l'inhomogénéité de chacun des paramètres de qualité de l'eau de l'échantillon ; $D_0$ représente un paramètre de diffusion de l'étape d'injection d'eau ; $D_1$ représente un paramètre de diffusion de l'étape stationnaire ; $D_2$ représente un paramètre de diffusion de l'étape de pompage de l'eau ; $v_0$ représente un paramètre de débit de l'étape d'injection d'eau. $v_2$ représente un paramètre de débit de l'étape de pompage d'eau, $v_0>0$, et $v_2<0$.

7. Dispositif électronique, **caractérisé en ce qu'**il comprend une mémoire, un processeur et un programme informatique stockés dans la mémoire et capables de fonctionner sur le processeur, dans lequel le processeur, lors de l'exécution du programme, implémente les étapes du procédé selon l'une des revendications 1 à 5.

8. Support de stockage lisible par ordinateur non transitoire, avec un programme informatique stocké à l'intérieur, **caractérisé en ce que** le programme, lorsqu'il est exécuté par un processeur, implémente les étapes du procédé selon l'une des revendications 1 à 5.

Determining first parameters, second parameters and third parameters of a to-be-controlled area $\quad\sim$ S101

Inputting the first parameters, the second parameters and the third parameters into a pumping-injection three-stage solute transport model to obtain fourth parameters output by the pumping-injection three-stage solute transport model $\quad\sim$ S102

Drawing up a control plan for the to-be-controlled area on the basis of the fourth parameters $\quad\sim$ S103

Fig. 1

Determining the third parameters on the basis of exploration data of the to-be-controlled area $\quad\sim$ S1011

Determining the first parameters and the second parameters $\quad\sim$ S1012

Fig. 2

Determining the sample area on the basis of the third parameters $\quad\sim$ A101

Determining the sample water quality parameters of the sample area $\quad\sim$ A102

Determining the first sample pumping-injection parameters $\quad\sim$ A103

Using the sample water quality parameters and the first sample pumping-injection parameters as parameters used for establishment to establish the pumping-injection three-stage solute transport model $\quad\sim$ A104

Fig. 3

Determining the sample area on the basis of the third parameters ~ A201

Determining the sample water quality parameters of the sample area ~ A202

Injecting a water tracer into the sample area, and determining the second sample pumping-injection parameters ~ A203

Using the sample water quality parameters and the second sample pumping-injection parameters as parameters used for establishment to establish the pumping-injection three-stage solute transport model ~ A204

Fig. 4

Determining the sample area on the basis of the third parameters ~ A301

Determining the sample water quality parameters and the working condition parameters of the sample area ~ A302

Determining the first sample pumping-injection parameters ~ A303

Using the sample water quality parameters, the first sample pumping-injection parameters and the working condition parameters as parameters used for establishment to establish the pumping-injection three-stage solute transport model ~ A304

Fig. 5

| Pulse fractional curve | $t_{push}$ (min) | Standing stage (min) | Water injection speed (m³h⁻¹) | Water pumping speed (m³h⁻¹) | Water injection volume (l) |
|---|---|---|---|---|---|
| CH3-1 | 14 | - | 0.6 | 0.53 | 139 |
| CH3-2 | 14 | 66 | 0.6 | 0.59 | 139 |
| CH3-3 | 55 | 69 | 0.59 | 0.59 | 544 |

Fig. 6

| Pulse fractional curve | $\alpha$ | $\beta$ (day$^{\alpha-1}$) | $\lambda$ ((10⁴day)⁻¹) | $v$ (m/day) | $D$ (m²/day) |
|---|---|---|---|---|---|
| CH3-CH12 | [0.58,0.6] | [1,1.5] | [0,0.1] | [18.8,4.8] | [1,1.5] |
| CH3-CH11 | [0.6,0.7] | [1,1] | [0,0.026] | [10,8] | [1,1.5] |
| CH10-CH11 | [0.55,0.62] | [1,1] | [0.04,0.02] | [3.7,2.5] | [3.5,3.5] |

Fig. 7

| Pulse fractional curve | $t_{push}$ (min) | Standing stage (min) | Water injection speed (m³h⁻¹) | Water pumping speed (m³h⁻¹) | Water injection volume (l) |
|---|---|---|---|---|---|
| CH10-1 | 14 | - | 0.52 | 0.53 | 121 |
| CH10-2 | 14 | 66 | 0.52 | 0.53 | 121 |
| CH10-3 | 55 | - | 0.52 | 0.53 | 477 |

Fig. 8

| Pulse fractional curve | $\alpha$ | $\beta$ (h$^{\alpha-1}$) | $\lambda$ ((h)⁻¹) | $v$ (m/h) | $D$ (m²/h) |
|---|---|---|---|---|---|
| CH10-1 | [0.83,0.83] | [1,1] | [0,1.6] | [9,-9.5] | [1.3,1] |
| CH10-2 | [0.83,0.83,0.83] | [1,1,1] | [0,0,0.7] | [9,0.2,-9.8] | [0.8,0.15,1] |
| CH10-3 | [0.83,0.83] | [1,1] | [0.7,0.15] | [8.8,-9.8] | [1.7,1.6] |

Fig. 9

Fig. 10

Fig. 11

| Parameter determination module | ~ 101 |

| Parameter input module | ~ 201 |

| Plan determination module | ~ 301 |

Fig. 12

Electronic device

510 Processor

530 Memory

Communication bus

540

520 Communication interface

Fig. 13

**EP 4 299 850 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 104671385 A